# EUROPEAN PATENT APPLICATION

(11) **EP 2 647 726 A1**
(43) Date of publication of application: **09.10.2013**
(21) Application number: 12163453.9
(22) Date of filing: 05.04.2012
(51) Int. Cl.: C12Q 1/68

(54) **Cardiovascular biomarkers**

(71) Applicant: Universitätsklinikum Freiburg, 79106 Freiburg (DE)
(72) Inventor: Walz, Gerd, 79104 Freiburg (DE); Schumacher, Martin, 79252 Stegen (DE); Kurz, Thorsten, 79114 Freiburg (DE); Binder, Harald, 55116 Mainz (DE)
(74) Representative: Kalhammer, Georg

(57) **Abstract**

The present invention relates to a method for predicting the risk of a cardiovascular event in a subject, comprising determining in a sample from said subject the expression level of at least one gene selected from the group consisting of PPBP, GP6, PF4, GP9, PDGFC and ITGA2B.

SEQUENCE LISTING

<110>  Universitaetsklinikum Freiburg

<120>  Cardiovascular Biomarkers

<130>  ZEE20090818

<160>  19

<170>  PatentIn version 3.5

<210>  1
<211>  1307
<212>  DNA
<213>  Homo sapiens

<400>  1
acttatctgc agacttgtag gcagcaactc accctcactc agaggtcttc tggttctgga      60

aacaactcta gctcagcctt ctccaccatg agcctcagac ttgataccac cccttcctgt     120

aacagtgcga gaccacttca tgccttgcag gtgctgctgc ttctgtcatt gctgctgact     180

gctctggctt cctccaccaa aggacaaact aagagaaact tggcgaaagg caaagaggaa     240

agtctagaca gtgacttgta tgctgaactc cgctgcatgt gtataaagac aacctctgga     300

attcatccca aaaacatcca aagtttggaa gtgatcggga aaggaaccca ttgcaaccaa     360

gtcgaagtga tagccacact gaaggatggg aggaaaatct gcctggaccc agatgctccc     420

agaatcaaga aaattgtaca gaaaaaattg gcaggtgatg aatctgctga ttaatttgtt     480

ctgtttctgc caaacttctt taactcccag gaagggtaga attttgaaac cttgattttc     540

tagagttctc atttattcag gatacctatt cttactgtat taaaatttgg atatgtgttt     600

cattctgtct caaaaatcac attttattct gagaaggttg gttaaaagat ggcagaaaga     660

agatgaaaat aaataagcct ggtttcaacc ctctaattct tgcctaaaca ttggactgta     720

ctttgcattt ttttctttaa aaatttctat tctaacacaa cttggttgat ttttcctggt     780

ctactttatg gttattagac atactcatgg gtattattag atttcataat ggtcaatgat     840

aataggaatt acatggagcc caacagagaa tatttgctca atacattttt gttaatatat     900

ttaggaactt aatggagtct ctcagtgtct tagtcctagg atgtcttatt taaaatactc     960

cctgaaagtt tattctgatg tttattttag ccatcaaaca ctaaaataat aaattggtga    1020

atatgaatct tataaactgt ggttagctgg tttaaagtga atatatttgc cactagtaga    1080

acaaaaatag atgatgaaaa tgaattaaca tatctacata gttataattc tatcattaga    1140

atgagcctta taaataagta caatatagga cttcaacctt actagactcc taattctaaa    1200

ttctactttt ttcatcaaca gaactttcat tcatttttta aaccctaaaa cttataccca    1260

cactattctt acaaaaatat tcacatgaaa taaaaatttg ctattga                 1307


<210>  2
<211>  2268

<212>  DNA
<213>  Homo sapiens

<400>  2
acagagctca ggacagggct gaggaaccat gtctccatcc ccgaccgccc tcttctgtct      60

tgggctgtgt ctggggcgtg tgccagcgca gagtggaccg ctccccaagc cctccctcca     120

ggctctgccc agctccctgg tgcccctgga gaagccagtg accctccggt gccagggacc     180

tccgggcgtg gacctgtacc gcctggagaa gctgagttcc agcaggtacc aggatcaggc     240

agtcctcttc atcccggcca tgaagagaag tctggctgga cgctaccgct gctcctacca     300

gaacggaagc ctctggtccc tgcccagcga ccagctggag ctcgttgcca cgggagtttt     360

tgccaaaccc tcgctctcag cccagcccgg cccggcggtg tcgtcaggag gggacgtaac     420

cctacagtgt cagactcggt atggctttga ccaatttgct ctgtacaagg aaggggaccc     480

tgcgccctac aagaatcccg agagatggta cagggctagt tttcccatca tcacggtgac     540

cgccgcccac agcggaacct accgatgcta cagcttctcc agcagggacc catacctgtg     600

gtcagccccc agcgaccccc tggagcttgt ggtcacagga acctctgtga cccccagccg     660

gttaccaaca gaaccacctt ccccggtagc agaattctca gaagccaccg ctgaactgac     720

cgtctcattc acaaacgaag tcttcacaac tgagacttct aggagtatca ccgccagtcc     780

aaaggagtca gactctccag ctggtgagtc ctgcccgcca gtactacacc aagggcaacc     840

tggtccggat atgcctcggg gctgtgatcc taataatcct ggcggggttt ctggcagagg     900

actggcacag ccggaggaag cgcctgcggc acaggggcag ggctgtgcag aggccgcttc     960

cgcccctccc gcccctcccg cagacccgga aatcacacgg gggtcaggat ggaggccgac    1020

aggatgttca cagccgcggg ttatgttcat gaccgctgaa ccccaggcac ggtcgtatcc    1080

aagggaggga tcatggcatg ggaggcgact caaagactgg cgtgtgtgga gcgtggaagc    1140

aggagggcag aggctacagc tgtggaaacg aggccatgct gcctcctcct ggtgttccat    1200

cagggagccg ttcggccagt gtctgtctgt ctgtctgcct ctctgtctga gggcaccctc    1260

catttgggat ggaaggaatc tgtggagacc ccatcctcct ccctgcacac tgtggatgac    1320

atggtaccct ggctggacca catactggcc tctttcttca acctctctaa tatgggctcc    1380

agacggatct ctaaggttcc cagctctcag ggttgactct gttccatcct ctgtgcaaaa    1440

tcctcccgtg cttccctttg gccctctgtg ctcttgtctg gttttcccca gaaactctca    1500

ccctcactcc atctcccact gcggtctaac aaatctcctt tcgtctctca gaacgggtct    1560

tgcaggcagt ttgggtatgt cattcatttt ccttagtgta aaactagcac gttgcccgct    1620

tcccttcaca ttagaaaaca agatcagcct gtgcaacatg gtgaaacctc atctctacca    1680

acaaaacaaa aaaacacaaa aattagccag gtgtggtggt gcatccctat actcccagca    1740

actcaggggg ctgaggtggg agaatggctt gagcctggga ggcagaggtt gcagtgagct    1800

gagatcacac cactgcactc tagctcgggt gacgaagcct gactttgtct caaaaaatac    1860

agggatgaat atgtcaatta ccctgatttg atcatagcac gttgtataca tgtactgcaa 1920

tattgctgtc caccccataa atatgtacaa ttctgtatac atttttaaaa tcataaaaat 1980

aagataatgc accgtctcca cccctctcat atttactttc tgaaggaaat gttaggtctt 2040

ctcaaggtaa agttctatat ttattatagc gtttaggcat ttcttgacca tctaatgagt 2100

gtaaaactgt accactgggc caagtgcagt ggatcatgtc tgtaatccta gcactgtggg 2160

aggccaaggc aggaggatcg cttgagccca ggagttcaag accagcctgg gcaacatagt 2220

gagaccccat ctctacttaa aataaagaag ataaaaattg ttttaaaa 2268

<210> 3
<211> 2264
<212> DNA
<213> Homo sapiens

<400> 3
acagagctca ggacagggct gaggaaccat gtctccatcc ccgaccgccc tcttctgtct 60

tgggctgtgt ctggggcgtg tgccagcgca gagtggaccg ctccccaagc cctccctcca 120

ggctctgccc agctccctgg tgcccctgga gaagccagtg accctccggt gccagggacc 180

tccgggcgtg gacctgtacc gcctggagaa gctgagttcc agcaggtacc aggatcaggc 240

agtcctcttc atcccggcca tgaagagaag tctggctgga cgctaccgct gctcctacca 300

gaacggaagc ctctggtccc tgcccagcga ccagctggag ctcgttgcca cgggagtttt 360

tgccaaaccc tcgctctcag cccagcccgg cccggcggtg tcgtcaggag gggacgtaac 420

cctacagtgt cagactcggt atggctttga ccaatttgct ctgtacaagg aaggggaccc 480

tgcgccctac aagaatcccg agagatggta cagggctagt tttcccatca tcacggtgac 540

cgccgcccac agcggaacct accgatgcta cagcttctcc agcagggacc catacctgtg 600

gtcagccccc agcgaccccc tggagcttgt ggtcacagga acctctgtga cccccagccg 660

gttaccaaca gaaccacctt ccccggtagc agaattctca gaagccaccg ctgaactgac 720

cgtctcattc acaaacgaag tcttcacaac tgagacttct aggagtatca ccgccagtcc 780

aaaggagtca gactctccag ctggtcctgc ccgccagtac tacaccaagg gcaacctggt 840

ccggatatgc ctcggggctg tgatcctaat aatcctggcg gggtttctgg cagaggactg 900

gcacagccgg aggaagcgcc tgcggcacag gggcagggct gtgcagaggc cgcttccgcc 960

cctcccgccc ctcccgcaga cccggaaatc acacgggggt caggatggag gccgacagga 1020

tgttcacagc cgcgggttat gttcatgacc gctgaacccc aggcacggtc gtatccaagg 1080

gagggatcat ggcatgggag gcgactcaaa gactggcgtg tgtggagcgt ggaagcagga 1140

gggcagaggc tacagctgtg gaaacgaggc catgctgcct cctcctggtg ttccatcagg 1200

gagccgttcg gccagtgtct gtctgtctgt ctgcctctct gtctgagggc accctccatt 1260

tgggatggaa ggaatctgtg gagaccccat cctcctccct gcacactgtg gatgacatgg 1320

taccctggct ggaccacata ctggcctctt tcttcaacct ctctaatatg ggctccagac 1380

ggatctctaa ggttcccagc tctcagggtt gactctgttc catcctctgt gcaaaatcct 1440

cccgtgcttc cctttggccc tctgtgctct tgtctggttt tccccagaaa ctctcaccct 1500

cactccatct cccactgcgg tctaacaaat ctcctttcgt ctctcagaac gggtcttgca 1560

ggcagtttgg gtatgtcatt cattttcctt agtgtaaaac tagcacgttg cccgcttccc 1620

ttcacattag aaaacaagat cagcctgtgc aacatggtga aacctcatct ctaccaacaa 1680

aacaaaaaaa cacaaaaatt agccaggtgt ggtggtgcat ccctatactc ccagcaactc 1740

agggggctga ggtgggagaa tggcttgagc ctgggaggca gaggttgcag tgagctgaga 1800

tcacaccact gcactctagc tcgggtgacg aagcctgact ttgtctcaaa aaatacaggg 1860

atgaatatgt caattaccct gatttgatca tagcacgttg tatacatgta ctgcaatatt 1920

gctgtccacc ccataaatat gtacaattct gtatacattt ttaaaatcat aaaaataaga 1980

taatgcaccg tctccacccc tctcatattt actttctgaa ggaaatgtta ggtcttctca 2040

aggtaaagtt ctatatttat tatagcgttt aggcatttct tgaccatcta atgagtgtaa 2100

aactgtacca ctgggccaag tgcagtggat catgtctgta atcctagcac tgtgggaggc 2160

caaggcagga ggatcgcttg agcccaggag ttcaagacca gcctgggcaa catagtgaga 2220

ccccatctct acttaaaata aagaagataa aaattgtttt aaaa 2264

<210> 4
<211> 2210
<212> DNA
<213> Homo sapiens

<400> 4
acagagctca ggacagggct gaggaaccat gtctccatcc ccgaccgccc tcttctgtct 60

tgggctgtgt ctggggcgtg tgccagcgca gagtggaccg ctccccaagc cctccctcca 120

ggctctgccc agctccctgg tgcccctgga gaagccagtg accctccggt gccagggacc 180

tccgggcgtg gacctgtacc gcctggagaa gctgagttcc agcaggtacc aggatcaggc 240

agtcctcttc atcccggcca tgaagagaag tctggctgga cgctaccgct gctcctacca 300

gaacggaagc ctctggtccc tgcccagcga ccagctggag ctcgttgcca cgggagtttt 360

tgccaaaccc tcgctctcag cccagcccgg cccggcggtg tcgtcaggag gggacgtaac 420

cctacagtgt cagactcggt atggctttga ccaatttgct ctgtacaagg aaggggaccc 480

tgcgccctac aagaatcccg agagatggta cagggctagt tttcccatca tcacggtgac 540

cgccgcccac agcggaacct accgatgcta cagcttctcc agcagggacc catacctgtg 600

gtcagccccc agcgaccccc tggagcttgt ggtcacagaa ttctcagaag ccaccgctga 660

actgaccgtc tcattcacaa acgaagtctt cacaactgag acttctagga gtatcaccgc 720

cagtccaaag gagtcagact ctccagctgg tcctgcccgc cagtactaca ccaagggcaa 780

cctggtccgg atatgcctcg gggctgtgat cctaataatc ctggcggggt ttctggcaga 840

ggactggcac agccggagga agcgcctgcg gcacaggggc agggctgtgc agaggccgct 900

tccgcccctc ccgcccctcc cgcagacccg gaaatcacac gggggtcagg atggaggccg 960

acaggatgtt cacagccgcg ggttatgttc atgaccgctg aaccccaggc acggtcgtat 1020

ccaagggagg gatcatggca tgggaggcga ctcaaagact ggcgtgtgtg gagcgtggaa 1080

gcaggagggc agaggctaca gctgtggaaa cgaggccatg ctgcctcctc ctggtgttcc 1140

atcagggagc cgttcggcca gtgtctgtct gtctgtctgc ctctctgtct gagggcaccc 1200

tccatttggg atggaaggaa tctgtggaga ccccatcctc ctccctgcac actgtggatg 1260

acatggtacc ctggctggac cacatactgg cctctttctt caacctctct aatatgggct 1320

ccagacggat ctctaaggtt cccagctctc agggttgact ctgttccatc ctctgtgcaa 1380

aatcctcccg tgcttccctt tggccctctg tgctcttgtc tggttttccc cagaaactct 1440

caccctcact ccatctccca ctgcggtcta acaaatctcc tttcgtctct cagaacgggt 1500

cttgcaggca gtttgggtat gtcattcatt ttccttagtg taaaactagc acgttgcccg 1560

cttcccttca cattagaaaa caagatcagc ctgtgcaaca tggtgaaacc tcatctctac 1620

caacaaaaca aaaaaacaca aaaattagcc aggtgtggtg gtgcatccct atactcccag 1680

caactcaggg ggctgaggtg ggagaatggc ttgagcctgg gaggcagagg ttgcagtgag 1740

ctgagatcac accactgcac tctagctcgg gtgacgaagc ctgactttgt ctcaaaaaat 1800

acagggatga atatgtcaat taccctgatt tgatcatagc acgttgtata catgtactgc 1860

aatattgctg tccaccccat aaatatgtac aattctgtat acatttttaa aatcataaaa 1920

ataagataat gcaccgtctc cacccctctc atatttactt tctgaaggaa atgttaggtc 1980

ttctcaaggt aaagttctat atttattata gcgtttaggc atttcttgac catctaatga 2040

gtgtaaaact gtaccactgg gccaagtgca gtggatcatg tctgtaatcc tagcactgtg 2100

ggaggccaag gcaggaggat cgcttgagcc caggagttca agaccagcct gggcaacata 2160

gtgagacccc atctctactt aaaataaaga agataaaaat tgttttaaaa 2210

<210> 5
<211> 858
<212> DNA
<213> Homo sapiens

<400> 5
gccaggacct ttcaggccag acaggagcac ctgaccaaag gcttcacagc cgccctcacc 60

gcccggcctt ctacggtgtc cagagacagt tagccaggcc tgggctgggc acactccacc 120

ttccctagtc accagctggt ttcccagagg agaaggctga gacccgagaa gggagccagc 180

ctgtcccatg cctgcctggg gagccctgtt cctgctctgg gccacagcag aggccaccaa 240

ggactgcccc agcccatgta cctgccgcgc cctggaaacc atggggctgt gggtggactg 300

caggggccac ggactcacgg ccctgcctgc cctgccggcc cgcacccgcc accttctgct 360

ggccaacaac agccttcagt ccgtgccccc gggagccttt gaccacctgc cccagctgca 420

gaccctcgat gtgacgcaga acccctggca ctgtgactgc agcctcacct atctgcgcct 480

ctggctggag gaccgcacgc ccgaggccct gctgcaggtc cgctgtgcca gccccagcct 540

cgctgcccat ggcccgctgg gccggctgac aggctaccag ctgggcagct gtggctggca 600

gctgcaggcg tcctgggtgc gcccgggggt cttgtgggac gtggcgctgg tcgccgtggc 660

cgcgctgggc ctggctcttc tggctggcct gctgtgtgcc accacagagg ccctggattg 720

agccaggccc ccagaacccc tggctccagg ccagggggcc agtccctgag gcaggtcccc 780

agactccacc aagcctggtc agcccaaacc accagaagcc cagaataaac tggcagctca 840

gctgttttat ataagctc 858

<210> 6
<211> 3018
<212> DNA
<213> Homo sapiens

<400> 6
gcccggagag ccgcatctat tggcagcttt gttattgatc agaaactgct cgccgccgac 60

ttggcttcca gtctggctgc gggcaaccct tgagttttcg cctctgtcct gtcccccgaa 120

ctgacaggtg ctcccagcaa cttgctgggg acttctcgcc gctcccccgc gtccccaccc 180

cctcattcct ccctcgcctt cacccccacc cccaccactt cgccacagct caggatttgt 240

ttaaaccttg ggaaactggt tcaggtccag gttttgcttt gatccttttc aaaaactgga 300

gacacagaag agggctctag gaaaaagttt tggatgggat tatgtggaaa ctaccctgcg 360

attctctgct gccagagcag gctcggcgct tccaccccag tgcagccttc ccctggcggt 420

ggtgaaagag actcgggagt cgctgcttcc aaagtgcccg ccgtgagtga gctctcaccc 480

cagtcagcca aatgagcctc ttcgggcttc tcctgctgac atctgccctg gccggccaga 540

gacaggggac tcaggcggaa tccaacctga gtagtaaatt ccagttttcc agcaacaagg 600

aacagaacgg agtacaagat cctcagcatg agagaattat tactgtgtct actaatggaa 660

gtattcacag cccaaggttt cctcatactt atccaagaaa tacggtcttg gtatggagat 720

tagtagcagt agaggaaaat gtatggatac aacttacgtt tgatgaaaga tttgggcttg 780

aagacccaga agatgacata tgcaagtatg attttgtaga agttgaggaa cccagtgatg 840

gaactatatt agggcgctgg tgtggttctg gtactgtacc aggaaaacag atttctaaag 900

gaaatcaaat taggataaga tttgtatctg atgaatattt tccttctgaa ccagggttct 960

gcatccacta caacattgtc atgccacaat tcacagaagc tgtgagtcct tcagtgctac 1020

ccccttcagc tttgccactg gacctgctta ataatgctat aactgccttt agtaccttgg 1080

aagaccttat tcgatatctt gaaccagaga gatggcagtt ggacttagaa gatctatata 1140

ggccaacttg gcaacttctt ggcaaggctt ttgtttttgg aagaaaatcc agagtggtgg 1200

atctgaacct tctaacagag gaggtaagat tatacagctg cacacctcgt aacttctcag 1260

tgtccataag ggaagaacta aagagaaccg ataccatttt ctggccaggt tgtctcctgg 1320

ttaaacgctg tggtgggaac tgtgcctgtt gtctccacaa ttgcaatgaa tgtcaatgtg 1380

tcccaagcaa agttactaaa aaataccacg aggtccttca gttgagacca aagaccggtg 1440

tcaggggatt gcacaaatca ctcaccgacg tggccctgga gcaccatgag gagtgtgact 1500

gtgtgtgcag agggagcaca ggaggatagc cgcatcacca ccagcagctc ttgcccagag 1560

ctgtgcagtg cagtggctga ttctattaga gaacgtatgc gttatctcca tccttaatct 1620

cagttgtttg cttcaaggac ctttcatctt caggatttac agtgcattct gaaagaggag 1680

acatcaaaca gaattaggag ttgtgcaaca gctcttttga gaggaggcct aaaggacagg 1740

agaaaaggtc ttcaatcgtg gaaagaaaat taaatgttgt attaaataga tcaccagcta 1800

gtttcagagt taccatgtac gtattccact agctgggttc tgtatttcag ttctttcgat 1860

acggcttagg gtaatgtcag tacaggaaaa aaactgtgca agtgagcacc tgattccgtt 1920

gccttgctta actctaaagc tccatgtcct gggcctaaaa tcgtataaaa tctggatttt 1980

tttttttttt tttgctcata ttcacatatg taaaccagaa cattctatgt actacaaacc 2040

tggtttttaa aaaggaacta tgttgctatg aattaaactt gtgtcgtgct gataggacag 2100

actggatttt tcatatttct tattaaaatt tctgccattt agaagaagag aactacattc 2160

atggtttgga agagataaac ctgaaaagaa gagtggcctt atcttcactt tatcgataag 2220

tcagtttatt tgtttcattg tgtacatttt tatattctcc ttttgacatt ataactgttg 2280

gcttttctaa tcttgttaaa tatatctatt tttaccaaag gtatttaata ttctttttta 2340

tgacaactta gatcaactat ttttagcttg gtaaattttt ctaaacacaa ttgttatagc 2400

cagaggaaca aagatgatat aaaatattgt tgctctgaca aaaatacatg tatttcattc 2460

tcgtatggtg ctagagttag attaatctgc attttaaaaa actgaattgg aatagaattg 2520

gtaagttgca aagacttttt gaaaataatt aaattatcat atcttccatt cctgttattg 2580

gagatgaaaa taaaaagcaa cttatgaaag tagacattca gatccagcca ttactaacct 2640

attccttttt tggggaaatc tgagcctagc tcagaaaaac ataaagcacc ttgaaaaaga 2700

cttggcagct tcctgataaa gcgtgctgtg ctgtgcagta ggaacacatc ctatttattg 2760

tgatgttgtg gttttattat cttaaactct gttccataca cttgtataaa tacatggata 2820

tttttatgta cagaagtatg tctcttaacc agttcactta ttgtactctg gcaatttaaa 2880

agaaaatcag taaaatattt tgcttgtaaa atgcttaata tcgtgcctag gttatgtggt 2940

gactatttga atcaaaaatg tattgaatca tcaaataaaa gaatgtggct attttgggga 3000

gaaaattaaa aaaaaaaa 3018

<210> 7
<211> 3334
<212> DNA
<213> Homo sapiens

<400> 7
attcctgcct gggaggttgt ggaagaagga agatggccag agctttgtgt ccactgcaag 60

ccctctggct tctggagtgg gtgctgctgc tcttgggacc ttgtgctgcc cctccagcct 120

gggccttgaa cctggaccca gtgcagctca ccttctatgc aggccccaat ggcagccagt 180

ttggattttc actggacttc cacaaggaca gccatgggag agtggccatc gtggtgggcg 240

ccccgcggac cctgggcccc agccaggagg agacgggcgg cgtgttcctg tgcccctgga 300

gggccgaggg cggccagtgc ccctcgctgc tctttgacct ccgtgatgag acccgaaatg 360

taggctccca aactttacaa accttcaagg cccgccaagg actgggggcg tcggtcgtca 420

gctggagcga cgtcattgtg gcctgcgccc cctggcagca ctggaacgtc ctagaaaaga 480

ctgaggaggc tgagaagacg cccgtaggta gctgcttttt ggctcagcca gagagcggcc 540

gccgcgccga gtactccccc tgtcgcggga acaccctgag ccgcatttac gtggaaaatg 600

attttagctg ggacaagcgt tactgtgaag cgggcttcag ctccgtggtc actcaggccg 660

gagagctggt gcttggggct cctggcggct attatttctt aggtctcctg gcccaggctc 720

cagttgcgga tattttctcg agttaccgcc caggcatcct tttgtggcac gtgtcctccc 780

agagcctctc ctttgactcc agcaacccag agtacttcga cggctactgg gggtactcgg 840

tggccgtggg cgagttcgac ggggatctca acactacaga atatgtcgtc ggtgccccca 900

cttggagctg gaccctggga gcggtggaaa ttttggattc ctactaccag aggctgcatc 960

ggctgcgcgg agagcagatg gcgtcgtatt ttgggcattc agtggctgtc actgacgtca 1020

acggggatgg gaggcatgat ctgctggtgg gcgctccact gtatatggag agccgggcag 1080

accgaaaact ggccgaagtg gggcgtgtgt atttgttcct gcagccgcga ggcccccacg 1140

cgctgggtgc ccccagcctc ctgctgactg gcacacagct ctatgggcga ttcggctctg 1200

ccatcgcacc cctgggcgac ctcgaccggg atggctacaa tgacattgca gtggctgccc 1260

cctacggggg tcccagtggc cggggccaag tgctggtgtt cctgggtcag agtgaggggc 1320

tgaggtcacg tccctcccag gtcctggaca gccccttccc cacaggctct gcctttggct 1380

tctcccttcg aggtgccgta gacatcgatg acaacggata cccagacctg atcgtgggag 1440

cttacggggc caaccaggtg gctgtgtaca gagctcagcc agtggtgaag gcctctgtcc 1500

agctactggt gcaagattca ctgaatcctg ctgtgaagag ctgtgtccta cctcagacca 1560

agacacccgt gagctgcttc aacatccaga tgtgtgttgg agccactggg cacaacattc 1620

ctcagaagct atccctaaat gccgagctgc agctggaccg gcagaagccc cgccagggcc 1680

ggcgggtgct gctgctgggc tctcaacagg caggcaccac cctgaacctg gatctgggcg 1740

gaaagcacag ccccatctgc cacaccacca tggccttcct tcgagatgag gcagacttcc 1800

gggacaagct gagccccatt gtgctcagcc tcaatgtgtc cctaccgccc acggaggctg 1860

gaatggcccc tgctgtcgtg ctgcatggag acacccatgt gcaggagcag acacgaatcg 1920

tcctggactg tggggaagat gacgtatgtg tgccccagct tcagctcact gccagcgtga 1980

cgggctcccc gctcctagtt ggggcagata atgtcctgga gctgcagatg gacgcagcca 2040

acgagggcga gggggcctat gaagcagagc tggccgtgca cctgccccag ggcgcccact 2100

acatgcgggc cctaagcaat gtcgagggct ttgagagact catctgtaat cagaagaagg 2160

agaatgagac cagggtggtg ctgtgtgagc tgggcaaccc catgaagaag aacgcccaga 2220

taggaatcgc gatgttggtg agcgtgggga atctggaaga ggctggggag tctgtgtcct 2280

tccagctgca gatacggagc aagaacagcc agaatccaaa cagcaagatt gtgctgctgg 2340

acgtgccggt ccgggcagag gcccaagtgg agctgcgagg gaactccttt ccagcctccc 2400

tggtggtggc agcagaagaa ggtgagaggg agcagaacag cttggacagc tggggaccca 2460

aagtggagca cacctatgag ctccacaaca atggccctgg gactgtgaat ggtcttcacc 2520

tcagcatcca ccttccggga cagtcccagc cctccgacct gctctacatc ctggatatac 2580

agccccaggg gggccttcag tgcttcccac agcctcctgt caaccctctc aaggtggact 2640

gggggctgcc catccccagc cctcccccca ttcacccggc ccatcacaag cgggatcgca 2700

gacagatctt cctgccagag cccgagcagc cctcgaggct tcaggatcca gttctcgtaa 2760

gctgcgactc ggcgccctgt actgtggtgc agtgtgacct gcaggagatg gcgcgcgggc 2820

agcgggccat ggtcacggtg ctggccttcc tgtggctgcc cagcctctac cagaggcctc 2880

tggatcagtt tgtgctgcag tcgcacgcat ggttcaacgt gtcctccctc ccctatgcgg 2940

tgcccccgct cagcctgccc cgaggggaag ctcaggtgtg gacacagctg ctccgggcct 3000

tggaggagag ggccattcca atctggtggg tgctggtggg tgtgctgggt ggcctgctgc 3060

tgctcaccat cctggtcctg gccatgtgga aggtcggctt cttcaagcgg aaccggccac 3120

ccctggaaga agatgatgaa gagggggagt gatggtgcag cctacactat tctagcagga 3180

gggttgggcg tgctacctgc accgcccctt ctccaacaag ttgcctccaa gctttgggtt 3240

ggagctgttc cattgggtcc tcttggtgtc gtttccctcc caacagagct gggctacccc 3300

ccctcctgct gcctaataaa gagactgagc cctg 3334

<210> 8
<211> 878
<212> DNA
<213> Homo sapiens

<400> 8
   atcttagttt ccgcaccgca gttcctcggt gtccacttca ggcttccgga ctggaaggac 60
   agccgggaat aaaacgtgcc ggcgaggctc aggagtcatt ggccacagag acccagcccg 120
   agtttcccat cgcactgagc actgagatcc tgctggaagc tctgccgcag catgagctcc 180
   gcagccgggt tctgcgcctc acgccccggg ctgctgttcc tggggttgct gctcctgcca 240
   cttgtggtcg ccttcgccag cgctgaagct gaagaagatg gggacctgca gtgcctgtgt 300
   gtgaagacca cctcccaggt ccgtcccagg cacatcacca gcctggaggt gatcaaggcc 360
   ggaccccact gccccactgc ccaactgata gccacgctga agaatggaag gaaaatttgc 420
   ttggacctgc aagccccgct gtacaagaaa ataattaaga aacttttgga gagttagcta 480
   ctagctgcct acgtgtgtgc atttgctata tagcatactt ctttttccca gtttcaatct 540
   aactgtgaaa gaacttctga tatttgtgtt atccttatga ttttaaataa acaaaataaa 600
   tcaagttgta gtatagtcaa aatacttctt aataatagtg caaaaattgt gttgacacat 660
   aacaatttca tggaagaaaa aaattccggt attttaagca aaaagtattt tgaaggaagg 720
   tgtgaatact ggttatgctt ggtgttacat gttggctgat acatattcat gcatttacat 780
   gattgcagta ctttatagct acatatttac cttgaccatt attattacct ttgccaataa 840
   atatcagtaa cacagaaaaa aaaaaaaaaa aaaaaaaa 878

<210> 9
<211> 94
<212> PRT
<213> Homo sapiens

<400> 9

Ser Ser Thr Lys Gly Gln Thr Lys Arg Asn Leu Ala Lys Gly Lys Glu
1               5                   10                  15

Glu Ser Leu Asp Ser Asp Leu Tyr Ala Glu Leu Arg Cys Met Cys Ile
            20                  25                  30

Lys Thr Thr Ser Gly Ile His Pro Lys Asn Ile Gln Ser Leu Glu Val
        35                  40                  45

Ile Gly Lys Gly Thr His Cys Asn Gln Val Glu Val Ile Ala Thr Leu
    50                  55                  60

Lys Asp Gly Arg Lys Ile Cys Leu Asp Pro Asp Ala Pro Arg Ile Lys
65                  70                  75                  80

Lys Ile Val Gln Lys Lys Leu Ala Gly Asp Glu Ser Ala Asp
                85                  90

<210> 10
<211> 85
<212> PRT
<213> Homo sapiens

<400> 10

Asn Leu Ala Lys Gly Lys Glu Glu Ser Leu Asp Ser Asp Leu Tyr Ala
1               5                   10                  15

Glu Leu Arg Cys Met Cys Ile Lys Thr Thr Ser Gly Ile His Pro Lys
            20                  25                  30

Asn Ile Gln Ser Leu Glu Val Ile Gly Lys Gly Thr His Cys Asn Gln
        35                  40                  45

Val Glu Val Ile Ala Thr Leu Lys Asp Gly Arg Lys Ile Cys Leu Asp
    50                  55                  60

Pro Asp Ala Pro Arg Ile Lys Lys Ile Val Gln Lys Lys Leu Ala Gly
65                  70                  75                  80

Asp Glu Ser Ala Asp
                85

<210>  11
<211>  81
<212>  PRT
<213>  Homo sapiens

<400>  11

Gly Lys Glu Glu Ser Leu Asp Ser Asp Leu Tyr Ala Glu Leu Arg Cys
1               5                   10                  15

Met Cys Ile Lys Thr Thr Ser Gly Ile His Pro Lys Asn Ile Gln Ser
            20                  25                  30

Leu Glu Val Ile Gly Lys Gly Thr His Cys Asn Gln Val Glu Val Ile
        35                  40                  45

Ala Thr Leu Lys Asp Gly Arg Lys Ile Cys Leu Asp Pro Asp Ala Pro
    50                  55                  60

Arg Ile Lys Lys Ile Val Gln Lys Lys Leu Ala Gly Asp Glu Ser Ala
65                  70                  75                  80

Asp

<210>  12
<211>  70
<212>  PRT
<213>  Homo sapiens

<400>  12

Ala Glu Leu Arg Cys Met Cys Ile Lys Thr Thr Ser Gly Ile His Pro
1               5                   10                  15

Lys Asn Ile Gln Ser Leu Glu Val Ile Gly Lys Gly Thr His Cys Asn
            20                  25                  30

Gln Val Glu Val Ile Ala Thr Leu Lys Asp Gly Arg Lys Ile Cys Leu
        35                  40                  45

Asp Pro Asp Ala Pro Arg Ile Lys Lys Ile Val Gln Lys Lys Leu Ala
    50                  55                  60

Gly Asp Glu Ser Ala Asp
65                  70

<210> 13
<211> 600
<212> PRT
<213> Homo sapiens

<400> 13

Gln Ser Gly Pro Leu Pro Lys Pro Ser Leu Gln Ala Leu Pro Ser Ser
1               5                   10                  15

Leu Val Pro Leu Glu Lys Pro Val Thr Leu Arg Cys Gln Gly Pro Pro
            20                  25                  30

Gly Val Asp Leu Tyr Arg Leu Glu Lys Leu Ser Ser Ser Arg Tyr Gln
        35                  40                  45

Asp Gln Ala Val Leu Phe Ile Pro Ala Met Lys Arg Ser Leu Ala Gly
    50                  55                  60

Arg Tyr Arg Cys Ser Tyr Gln Asn Gly Ser Leu Trp Ser Leu Pro Ser
65                  70                  75                  80

Asp Gln Leu Glu Leu Val Ala Thr Gly Val Phe Ala Lys Pro Ser Leu
                85                  90                  95

Ser Ala Gln Pro Gly Pro Ala Val Ser Ser Gly Gly Asp Val Thr Leu
            100                 105                 110

Gln Cys Gln Thr Arg Tyr Gly Phe Asp Gln Phe Ala Leu Tyr Lys Glu
        115                 120                 125

Gly Asp Pro Ala Pro Tyr Lys Asn Pro Glu Arg Trp Tyr Arg Ala Ser
    130                 135                 140

Phe Pro Ile Ile Thr Val Thr Ala Ala His Ser Gly Thr Tyr Arg Cys
145                 150                 155                 160

Tyr Ser Phe Ser Ser Arg Asp Pro Tyr Leu Trp Ser Ala Pro Ser Asp

                165                 170                 175

Pro Leu Glu Leu Val Val Thr Gly Thr Ser Val Thr Pro Ser Arg Leu
            180                 185                 190

Pro Thr Glu Pro Pro Ser Pro Val Ala Glu Phe Ser Glu Ala Thr Ala
        195                 200                 205

Glu Leu Thr Val Ser Phe Thr Asn Glu Val Phe Thr Thr Glu Thr Ser
    210                 215                 220

Arg Ser Ile Thr Ala Ser Pro Lys Glu Ser Asp Ser Pro Ala Gly Glu
225                 230                 235                 240

Ser Cys Pro Pro Val Leu His Gln Gly Gln Pro Gly Pro Asp Met Pro
                245                 250                 255

Arg Gly Cys Asp Pro Asn Asn Pro Gly Gly Val Ser Gly Arg Gly Leu
            260                 265                 270

Ala Gln Pro Glu Glu Ala Pro Ala Ala Gln Gly Gln Gly Cys Ala Glu
        275                 280                 285

Ala Ala Ser Ala Pro Pro Ala Pro Pro Ala Asp Pro Glu Ile Thr Arg
    290                 295                 300

Gly Ser Gly Trp Arg Pro Thr Gly Cys Ser Gln Pro Arg Val Met Phe
305                 310                 315                 320

Met Thr Ala Glu Pro Gln Ala Arg Ser Tyr Pro Arg Glu Gly Ser Trp
                325                 330                 335

His Gly Arg Arg Leu Lys Asp Trp Arg Val Trp Ser Val Glu Ala Gly
            340                 345                 350

Gly Gln Arg Leu Gln Leu Trp Lys Arg Gly His Ala Ala Ser Ser Trp
        355                 360                 365

Cys Ser Ile Arg Glu Pro Phe Gly Gln Cys Leu Ser Val Cys Leu Pro
    370                 375                 380

Leu Cys Leu Arg Ala Pro Ser Ile Trp Asp Gly Arg Asn Leu Trp Arg
385                 390                 395                 400

Pro His Pro Pro Pro Cys Thr Leu Trp Met Thr Trp Tyr Pro Gly Trp
                405                 410                 415

Thr Thr Tyr Trp Pro Leu Ser Ser Thr Ser Leu Ile Trp Ala Pro Asp
            420                 425                 430

Gly Ser Leu Arg Phe Pro Ala Leu Arg Val Asp Ser Val Pro Ser Ser
        435                 440                 445

Val Gln Asn Pro Pro Val Leu Pro Phe Gly Pro Leu Cys Ser Cys Leu
    450                 455                 460

Val Phe Pro Arg Asn Ser His Pro His Ser Ile Ser His Cys Gly Leu
465                 470                 475                 480

Thr Asn Leu Leu Ser Ser Leu Arg Thr Gly Leu Ala Gly Ser Leu Gly
                485                 490                 495

Met Ser Phe Ile Phe Leu Ser Val Lys Leu Ala Arg Cys Pro Leu Pro
            500                 505                 510

Phe Thr Leu Glu Asn Lys Ile Ser Leu Cys Asn Met Val Lys Pro His
        515                 520                 525

Leu Tyr Gln Gln Asn Lys Lys Thr Gln Lys Leu Ala Arg Cys Gly Gly
    530                 535                 540

Ala Ser Leu Tyr Ser Gln Gln Leu Arg Gly Leu Arg Trp Glu Asn Gly
545                 550                 555                 560

Leu Ser Leu Gly Gly Arg Gly Cys Ser Glu Leu Arg Ser His His Cys
                565                 570                 575

Thr Leu Ala Arg Val Thr Lys Pro Asp Phe Val Ser Lys Asn Thr Gly
            580                 585                 590

Met Asn Met Ser Ile Thr Leu Ile
        595                 600

<210>  14
<211>  319
<212>  PRT
<213>  Homo sapiens

<400>  14

Gln Ser Gly Pro Leu Pro Lys Pro Ser Leu Gln Ala Leu Pro Ser Ser
1               5                   10                  15

Leu Val Pro Leu Glu Lys Pro Val Thr Leu Arg Cys Gln Gly Pro Pro
            20                  25                  30

Gly Val Asp Leu Tyr Arg Leu Glu Lys Leu Ser Ser Ser Arg Tyr Gln
        35                  40                  45

Asp Gln Ala Val Leu Phe Ile Pro Ala Met Lys Arg Ser Leu Ala Gly
    50                  55                  60

Arg Tyr Arg Cys Ser Tyr Gln Asn Gly Ser Leu Trp Ser Leu Pro Ser
65                  70                  75                  80

Asp Gln Leu Glu Leu Val Ala Thr Gly Val Phe Ala Lys Pro Ser Leu
                85                  90                  95

Ser Ala Gln Pro Gly Pro Ala Val Ser Ser Gly Gly Asp Val Thr Leu
            100                 105                 110

Gln Cys Gln Thr Arg Tyr Gly Phe Asp Gln Phe Ala Leu Tyr Lys Glu
        115                 120                 125

Gly Asp Pro Ala Pro Tyr Lys Asn Pro Glu Arg Trp Tyr Arg Ala Ser
    130                 135                 140

Phe Pro Ile Ile Thr Val Thr Ala Ala His Ser Gly Thr Tyr Arg Cys
145                 150                 155                 160

Tyr Ser Phe Ser Ser Arg Asp Pro Tyr Leu Trp Ser Ala Pro Ser Asp
                165                 170                 175

Pro Leu Glu Leu Val Val Thr Gly Thr Ser Val Thr Pro Ser Arg Leu
            180                 185                 190

Pro Thr Glu Pro Pro Ser Pro Val Ala Glu Phe Ser Glu Ala Thr Ala
        195                 200                 205

Glu Leu Thr Val Ser Phe Thr Asn Glu Val Phe Thr Thr Glu Thr Ser
    210                 215                 220

Arg Ser Ile Thr Ala Ser Pro Lys Glu Ser Asp Ser Pro Ala Gly Pro
225                 230                 235                 240

Ala Arg Gln Tyr Tyr Thr Lys Gly Asn Leu Val Arg Ile Cys Leu Gly
                245                 250                 255

Ala Val Ile Leu Ile Ile Leu Ala Gly Phe Leu Ala Glu Asp Trp His
            260                 265                 270

Ser Arg Arg Lys Arg Leu Arg His Arg Gly Arg Ala Val Gln Arg Pro
        275                 280                 285

Leu Pro Pro Leu Pro Pro Leu Pro Gln Thr Arg Lys Ser His Gly Gly
    290                 295                 300

Gln Asp Gly Gly Arg Gln Asp Val His Ser Arg Gly Leu Cys Ser

305                 310                 315

<210>  15
<211>  301
<212>  PRT
<213>  Homo sapiens

<400>  15

Gln Ser Gly Pro Leu Pro Lys Pro Ser Leu Gln Ala Leu Pro Ser Ser
1               5                   10                  15

Leu Val Pro Leu Glu Lys Pro Val Thr Leu Arg Cys Gln Gly Pro Pro
            20                  25                  30

Gly Val Asp Leu Tyr Arg Leu Glu Lys Leu Ser Ser Ser Arg Tyr Gln
        35                  40                  45

Asp Gln Ala Val Leu Phe Ile Pro Ala Met Lys Arg Ser Leu Ala Gly
    50                  55                  60

Arg Tyr Arg Cys Ser Tyr Gln Asn Gly Ser Leu Trp Ser Leu Pro Ser
65                  70                  75                  80

Asp Gln Leu Glu Leu Val Ala Thr Gly Val Phe Ala Lys Pro Ser Leu
                85                  90                  95

Ser Ala Gln Pro Gly Pro Ala Val Ser Ser Gly Gly Asp Val Thr Leu
            100                 105                 110

Gln Cys Gln Thr Arg Tyr Gly Phe Asp Gln Phe Ala Leu Tyr Lys Glu
        115                 120                 125

Gly Asp Pro Ala Pro Tyr Lys Asn Pro Glu Arg Trp Tyr Arg Ala Ser
    130                 135                 140

Phe Pro Ile Ile Thr Val Thr Ala Ala His Ser Gly Thr Tyr Arg Cys
145                 150                 155                 160

Tyr Ser Phe Ser Ser Arg Asp Pro Tyr Leu Trp Ser Ala Pro Ser Asp
                165                 170                 175

Pro Leu Glu Leu Val Val Thr Glu Phe Ser Glu Ala Thr Ala Glu Leu
            180                 185                 190

Thr Val Ser Phe Thr Asn Glu Val Phe Thr Thr Glu Thr Ser Arg Ser
        195                 200                 205

Ile Thr Ala Ser Pro Lys Glu Ser Asp Ser Pro Ala Gly Pro Ala Arg
    210                 215                 220

Gln Tyr Tyr Thr Lys Gly Asn Leu Val Arg Ile Cys Leu Gly Ala Val
225                 230                 235                 240

Ile Leu Ile Ile Leu Ala Gly Phe Leu Ala Glu Asp Trp His Ser Arg
                245                 250                 255

Arg Lys Arg Leu Arg His Arg Gly Arg Ala Val Gln Arg Pro Leu Pro
            260                 265                 270

Pro Leu Pro Pro Leu Pro Gln Thr Arg Lys Ser His Gly Gly Gln Asp
        275                 280                 285

Gly Gly Arg Gln Asp Val His Ser Arg Gly Leu Cys Ser
    290                 295                 300

<210>  16
<211>  161
<212>  PRT
<213>  Homo sapiens

<400>  16

Thr Lys Asp Cys Pro Ser Pro Cys Thr Cys Arg Ala Leu Glu Thr Met
1               5                   10                  15

Gly Leu Trp Val Asp Cys Arg Gly His Gly Leu Thr Ala Leu Pro Ala
            20                  25                  30

Leu Pro Ala Arg Thr Arg His Leu Leu Leu Ala Asn Asn Ser Leu Gln
        35                  40                  45

Ser Val Pro Pro Gly Ala Phe Asp His Leu Pro Gln Leu Gln Thr Leu
    50                  55                  60

Asp Val Thr Gln Asn Pro Trp His Cys Asp Cys Ser Leu Thr Tyr Leu
65                  70                  75                  80

Arg Leu Trp Leu Glu Asp Arg Thr Pro Glu Ala Leu Leu Gln Val Arg
                85                  90                  95

Cys Ala Ser Pro Ser Leu Ala Ala His Gly Pro Leu Gly Arg Leu Thr
            100                 105                 110

Gly Tyr Gln Leu Gly Ser Cys Gly Trp Gln Leu Gln Ala Ser Trp Val
        115                 120                 125

Arg Pro Gly Val Leu Trp Asp Val Ala Leu Val Ala Val Ala Ala Leu
    130                 135                 140

Gly Leu Ala Leu Leu Ala Gly Leu Leu Cys Ala Thr Thr Glu Ala Leu

145                 150                 155                 160

Asp

<210>  17
<211>  323
<212>  PRT
<213>  Homo sapiens

<400>  17

Glu Ser Asn Leu Ser Ser Lys Phe Gln Phe Ser Ser Asn Lys Glu Gln
1               5                   10                  15

Asn Gly Val Gln Asp Pro Gln His Glu Arg Ile Ile Thr Val Ser Thr
            20                  25                  30

Asn Gly Ser Ile His Ser Pro Arg Phe Pro His Thr Tyr Pro Arg Asn
        35                  40                  45

Thr Val Leu Val Trp Arg Leu Val Ala Val Glu Glu Asn Val Trp Ile
    50                  55                  60

Gln Leu Thr Phe Asp Glu Arg Phe Gly Leu Glu Asp Pro Glu Asp Asp
65                  70                  75                  80

Ile Cys Lys Tyr Asp Phe Val Glu Val Glu Glu Pro Ser Asp Gly Thr
                85                  90                  95

Ile Leu Gly Arg Trp Cys Gly Ser Gly Thr Val Pro Gly Lys Gln Ile
            100                 105                 110

Ser Lys Gly Asn Gln Ile Arg Ile Arg Phe Val Ser Asp Glu Tyr Phe
        115                 120                 125

Pro Ser Glu Pro Gly Phe Cys Ile His Tyr Asn Ile Val Met Pro Gln
    130                 135                 140

Phe Thr Glu Ala Val Ser Pro Ser Val Leu Pro Pro Ser Ala Leu Pro
145                 150                 155                 160

Leu Asp Leu Leu Asn Asn Ala Ile Thr Ala Phe Ser Thr Leu Glu Asp
                165                 170                 175

Leu Ile Arg Tyr Leu Glu Pro Glu Arg Trp Gln Leu Asp Leu Glu Asp
            180                 185                 190

Leu Tyr Arg Pro Thr Trp Gln Leu Leu Gly Lys Ala Phe Val Phe Gly
        195                 200                 205

Arg Lys Ser Arg Val Val Asp Leu Asn Leu Leu Thr Glu Glu Val Arg
    210                 215                 220

Leu Tyr Ser Cys Thr Pro Arg Asn Phe Ser Val Ser Ile Arg Glu Glu
225                 230                 235                 240

Leu Lys Arg Thr Asp Thr Ile Phe Trp Pro Gly Cys Leu Leu Val Lys
                245                 250                 255

Arg Cys Gly Gly Asn Cys Ala Cys Cys Leu His Asn Cys Asn Glu Cys
            260                 265                 270

Gln Cys Val Pro Ser Lys Val Thr Lys Lys Tyr His Glu Val Leu Gln
        275                 280                 285

Leu Arg Pro Lys Thr Gly Val Arg Gly Leu His Lys Ser Leu Thr Asp
    290                 295                 300

Val Ala Leu Glu His His Glu Glu Cys Asp Cys Val Cys Arg Gly Ser
305                 310                 315                 320

Thr Gly Gly

<210>  18
<211>  871
<212>  PRT
<213>  Homo sapiens

<400>  18

Leu Asn Leu Asp Pro Val Gln Leu Thr Phe Tyr Ala Gly Pro Asn Gly
1               5                   10                  15

Ser Gln Phe Gly Phe Ser Leu Asp Phe His Lys Asp Ser His Gly Arg
            20                  25                  30

Val Ala Ile Val Val Gly Ala Pro Arg Thr Leu Gly Pro Ser Gln Glu
        35                  40                  45

Glu Thr Gly Gly Val Phe Leu Cys Pro Trp Arg Ala Glu Gly Gly Gln
    50                  55                  60

Cys Pro Ser Leu Leu Phe Asp Leu Arg Asp Glu Thr Arg Asn Val Gly
65                  70                  75                  80

Ser Gln Thr Leu Gln Thr Phe Lys Ala Arg Gln Gly Leu Gly Ala Ser
                85                  90                  95

Val Val Ser Trp Ser Asp Val Ile Val Ala Cys Ala Pro Trp Gln His

            100                 105                 110

Trp Asn Val Leu Glu Lys Thr Glu Glu Ala Glu Lys Thr Pro Val Gly
        115                 120                 125

Ser Cys Phe Leu Ala Gln Pro Glu Ser Gly Arg Arg Ala Glu Tyr Ser
    130                 135                 140

Pro Cys Arg Gly Asn Thr Leu Ser Arg Ile Tyr Val Glu Asn Asp Phe
145                 150                 155                 160

Ser Trp Asp Lys Arg Tyr Cys Glu Ala Gly Phe Ser Ser Val Val Thr
                165                 170                 175

Gln Ala Gly Glu Leu Val Leu Gly Ala Pro Gly Gly Tyr Tyr Phe Leu
            180                 185                 190

Gly Leu Leu Ala Gln Ala Pro Val Ala Asp Ile Phe Ser Ser Tyr Arg
        195                 200                 205

Pro Gly Ile Leu Leu Trp His Val Ser Ser Gln Ser Leu Ser Phe Asp
    210                 215                 220

Ser Ser Asn Pro Glu Tyr Phe Asp Gly Tyr Trp Gly Tyr Ser Val Ala
225                 230                 235                 240

Val Gly Glu Phe Asp Gly Asp Leu Asn Thr Thr Glu Tyr Val Val Gly
                245                 250                 255

Ala Pro Thr Trp Ser Trp Thr Leu Gly Ala Val Glu Ile Leu Asp Ser
            260                 265                 270

Tyr Tyr Gln Arg Leu His Arg Leu Arg Gly Glu Gln Met Ala Ser Tyr
        275                 280                 285

Phe Gly His Ser Val Ala Val Thr Asp Val Asn Gly Asp Gly Arg His
    290                 295                 300

Asp Leu Leu Val Gly Ala Pro Leu Tyr Met Glu Ser Arg Ala Asp Arg
305                 310                 315                 320

Lys Leu Ala Glu Val Gly Arg Val Tyr Leu Phe Leu Gln Pro Arg Gly
                325                 330                 335

Pro His Ala Leu Gly Ala Pro Ser Leu Leu Leu Thr Gly Thr Gln Leu
            340                 345                 350

Tyr Gly Arg Phe Gly Ser Ala Ile Ala Pro Leu Gly Asp Leu Asp Arg
        355                 360                 365

Asp Gly Tyr Asn Asp Ile Ala Val Ala Ala Pro Tyr Gly Gly Pro Ser
    370                 375                 380

Gly Arg Gly Gln Val Leu Val Phe Leu Gly Gln Ser Glu Gly Leu Arg
385                 390                 395                 400

Ser Arg Pro Ser Gln Val Leu Asp Ser Pro Phe Pro Thr Gly Ser Ala
                405                 410                 415

Phe Gly Phe Ser Leu Arg Gly Ala Val Asp Ile Asp Asp Asn Gly Tyr
            420                 425                 430

Pro Asp Leu Ile Val Gly Ala Tyr Gly Ala Asn Gln Val Ala Val Tyr
        435                 440                 445

Arg Ala Gln Pro Val Val Lys Ala Ser Val Gln Leu Leu Val Gln Asp
    450                 455                 460

Ser Leu Asn Pro Ala Val Lys Ser Cys Val Leu Pro Gln Thr Lys Thr
465                 470                 475                 480

Pro Val Ser Cys Phe Asn Ile Gln Met Cys Val Gly Ala Thr Gly His
                485                 490                 495

Asn Ile Pro Gln Lys Leu Ser Leu Asn Ala Glu Leu Gln Leu Asp Arg
            500                 505                 510

Gln Lys Pro Arg Gln Gly Arg Arg Val Leu Leu Leu Gly Ser Gln Gln
        515                 520                 525

Ala Gly Thr Thr Leu Asn Leu Asp Leu Gly Gly Lys His Ser Pro Ile
    530                 535                 540

Cys His Thr Thr Met Ala Phe Leu Arg Asp Glu Ala Asp Phe Arg Asp
545                 550                 555                 560

Lys Leu Ser Pro Ile Val Leu Ser Leu Asn Val Ser Leu Pro Pro Thr
                565                 570                 575

Glu Ala Gly Met Ala Pro Ala Val Val Leu His Gly Asp Thr His Val
            580                 585                 590

Gln Glu Gln Thr Arg Ile Val Leu Asp Cys Gly Glu Asp Asp Val Cys
        595                 600                 605

Val Pro Gln Leu Gln Leu Thr Ala Ser Val Thr Gly Ser Pro Leu Leu
    610                 615                 620

Val Gly Ala Asp Asn Val Leu Glu Leu Gln Met Asp Ala Ala Asn Glu
625                 630                 635                 640

Gly Glu Gly Ala Tyr Glu Ala Glu Leu Ala Val His Leu Pro Gln Gly
                645                 650                 655

Ala His Tyr Met Arg Ala Leu Ser Asn Val Glu Gly Phe Glu Arg Leu
            660                 665                 670

Ile Cys Asn Gln Lys Lys Glu Asn Glu Thr Arg Val Val Leu Cys Glu
        675                 680                 685

Leu Gly Asn Pro Met Lys Lys Asn Ala Gln Ile Gly Ile Ala Met Leu
    690                 695                 700

Val Ser Val Gly Asn Leu Glu Glu Ala Gly Glu Ser Val Ser Phe Gln
705                 710                 715                 720

Leu Gln Ile Arg Ser Lys Asn Ser Gln Asn Pro Asn Ser Lys Ile Val
                725                 730                 735

Leu Leu Asp Val Pro Val Arg Ala Glu Ala Gln Val Glu Leu Arg Gly
            740                 745                 750

Asn Ser Phe Pro Ala Ser Leu Val Val Ala Ala Glu Glu Gly Glu Arg
        755                 760                 765

Glu Gln Asn Ser Leu Asp Ser Trp Gly Pro Lys Val Glu His Thr Tyr
    770                 775                 780

Glu Leu His Asn Asn Gly Pro Gly Thr Val Asn Gly Leu His Leu Ser
785                 790                 795                 800

Ile His Leu Pro Gly Gln Ser Gln Pro Ser Asp Leu Leu Tyr Ile Leu
                805                 810                 815

Asp Ile Gln Pro Gln Gly Gly Leu Gln Cys Phe Pro Gln Pro Pro Val
            820                 825                 830

Asn Pro Leu Lys Val Asp Trp Gly Leu Pro Ile Pro Ser Pro Ser Pro
        835                 840                 845

Ile His Pro Ala His His Lys Arg Asp Arg Arg Gln Ile Phe Leu Pro
    850                 855                 860

Glu Pro Glu Gln Pro Ser Arg
865                 870

<210>  19
<211>  70
<212>  PRT
<213>  Homo sapiens

<400>  19

Glu Ala Glu Glu Asp Gly Asp Leu Gln Cys Leu Cys Val Lys Thr Thr
1               5                   10                  15

Ser Gln Val Arg Pro Arg His Ile Thr Ser Leu Glu Val Ile Lys Ala
            20                  25                  30

Gly Pro His Cys Pro Thr Ala Gln Leu Ile Ala Thr Leu Lys Asn Gly
        35                  40                  45

Arg Lys Ile Cys Leu Asp Leu Gln Ala Pro Leu Tyr Lys Lys Ile Ile
    50                  55                  60

Lys Lys Leu Leu Glu Ser
65                  70

## Description

The present invention concerns a method for predicting the risk of a cardiovascular event in a patient with end-stage renal disease treated with intermittent hemodialysis.

### BACKGROUND OF THE INVENTION

Cardiovascular diseases are one of the leading causes of death worldwide. For example, 1.5 million Americans suffer a myocardial infarction as a major cardiovascular event every year and half a million die.

Biomarkers that are measurable and quantifiable biological parameters serve as valuable tools for the assessment of pathophysiological conditions, such as myocardial infarction. A biomarker that is used clinically in the diagnosis of acute myocardial infarction is troponin, which is released from myocytes after irreversible myocardial damage has occurred. US 2005/0250156 A1 furthermore envisaged the use of platelet activation markers, inflammatory markers and coagulation markers in the assessment of acute myocardial infarction or drug resistance. Xu et al aimed to define biomarkers for the etiology of ischemic stroke. They found platelet-derived hemostasis genes to be significantly regulated in atherosclerotic but not cardioembolic stroke patients (Xu H et al, J Cereb Blood Flow Metab. 2008 Jul;28(7):1320-8*).*

Several studies have established an association between chronic kidney disease and cardiovascular disease, including sudden death, stroke, congestive heart failure and myocardial infarction (Anavekar NS et al, N Engl J Med. 2004;351:1285-1295). A high risk for cardiovascular events seems to be present for patients with end-stage renal disease (ESRD), particularly if the patient is receiving renal replacement therapy. Cardiovascular disease in ESRD patients has largely been attributed to the significant co-morbidity such as advanced age, hypertension and diabetes mellitus, i.e. conditions often associated with both chronic renal failure and cardiovascular disease. However, these clinical risk factors alone cannot explain the high incidence of cardiovascular events in ESRD and particularly younger ESRD patients.

Once patients reach end-stage renal disease and require renal replacement therapy, the yearly mortality exceeds 15-20% (Weinhandl ED et al, J Am Soc Nephrol. 2010;21:499-506). More than 50% of ESRD patient death are caused by cardiovascular events (Herzog CA et al, N Engl J Med. 1998;339:799-805; Himmelfarb J, Curr Opin Nephrol Hypertens. 2003;12:587-591) and the two-year mortality rate after myocardial infarction among patients suffering from ESRD is twice the mortality rate compared to myocardial infarction in the general population (Sarnak MJ et al, Hypertension. 2003;42:1050-1065*).* It is also referred to an "accelerated vascular disease" in ESRD patients.

Interestingly, some of the conventional treatments of cardiovascular disease are not or not as effective in ESRD patients as in other patients. For example, lipid-lowering drugs, which are typically powerful agents to combat cardiovascular disease completely failed to improve survival in patients undergoing hemodialysis (Fellstrom BC et al, N Engl J Med. 2009;360:1395-1407*;* Wanner C et al, N Engl J Med. 2005;353:238-248), which is the most commonly utilized treatment modality for patients with end-stage renal disease. Moreover, anticoagulation and platelet inhibitors negatively affect survival of hemodialysis patients. In a large meta-analysis, patients exposed to warfarin, clopidogrel, and/or aspirin had significantly increased mortality rates (Chan KE et al, J Am Soc Nephrol. 2009;20:2223-2233; Chan KE et al, J Am Soc Nephrol. 2009;20:872-881).

These observations suggest that the prevalence of cardiovascular diseases is only partly explained by conventional risk factors.

Therefore, determination of biomarkers in ESRD patients that are predictive for the risk of a cardiovascular event are highly desirable and could be used to identify ESRD patients who might profit from e.g. a closer monitoring for future cardiovascular events and/or early treatment.

IA study that prospectively followed hemodialysis (the most common renal replacement therapy for ESRD) patients, an increased cardiovascular mortality was reported for patients having elevated platelet factor 4 (PF4)-heparin antibodies (Pena de la Vega L et al, Mayo Clin Proc. 2005 Aug;80(8):995-1000*).* The antibody recognizes complexes formed between heparin and endogenous PF4 causing potentially life threatening heparin-induced thrombocytopenia. The study focused on the PF4-heparin antibody as a marker for increased cardiovascular mortality; no correlation between expression of PF4 and cardiovascular events was established.

Ashman et al studied platelet activation and circulating platelet-leucocyte aggregates and found that platelet-monocyte aggregation was associated with increased cardiovascular events in a small study of 20 ESRD patients (Ashman N et al, Nephrol Dial Transplant. 2003 Oct;18(10):2088-96*).* This may suggest a predictive value of certain cell aggregates for a cardiovascular event. However, no correlation between cardiovascular events and increased expression of certain genes, particularly platelet genes, was made.

WO 2011/000874 A1 claims the circulating levels of endothelial microparticles as a predictive marker for the cardiovascular mortality risk of a patient. The suitability of the marker was based on the analysis of plasma samples of hemodialyzed ESRD patients. Patients with baseline level of endothelial microparticles above media were reported to have a higher incidence of all-cause and cardiovascular death. This conclusion was not reached for plasma microparticles from other cellular origin as e.g. assessed for microparticles from platelets.

There is a need for the provision of suitable biomarkers on nucleic acid or protein level useful in the prediction of the risk of a patient to suffer from a cardiovascular event or cardiovascular mortality or to allow monitoring the impact of a treatment or cardiovascular disease progression.

### SUMMARY OF THE INVENTION

In a first aspect, the present invention relates to a method for predicting the risk of a cardiovascular event in a subject suffering from end-stage renal disease, comprising determining in a sample from said subject the expression level of at least one gene selected from the group consisting of PPBP, GP6, PF4, GP9, PDGFC and ITGA2B. Preferably, the method comprises determining the expression of at least one mRNA-biomarker or at least one protein-biomarker selected from the group of translational products of said mRNAs in a sample from said subject.

In a second aspect, the present invention relates to a method for predicting the mortality risk after a cardiovascular event in a subject suffering from end-stage renal disease, comprising determining in a sample from said subject the expression level of at least one gene selected from the group consisting of PPBP, GP6, PF4, GP9, PDGFC and ITGA2B. Preferably, the method comprises determining the expression of at least one mRNA-biomarker or at least one protein-biomarker selected from the group of translational products of said mRNAs in a sample from said subject.

In a third aspect, the present invention relates to a method for monitoring the impact of a treatment administered to a subject suffering from end-stage renal disease on the risk of a cardiovascular event, comprising determining in a sample from said subject the expression level of at least one gene selected from the group consisting of PPBP, GP6, PF4, GP9, PDGFC and ITGA2B. Preferably, the method comprises determining the expression of at least one mRNA-biomarker or at least one protein-biomarker selected from the group of translational products of said mRNAs in a sample from said subject.

In another aspect, the present invention relates to a method for predicting a cardiovascular disease or for monitoring the progression of a cardiovascular disease in a subject suffering from end-stage renal disease, comprising determining in a sample from said subject the expression level of at least one gene selected from the group consisting of PPBP, GP6, PF4, GP9, PDGFC and ITGA2B. Preferably, the method comprises determining the expression of at least one mRNA-biomarker or at least one protein-biomarker selected from the group of translational products of said mRNAs in a sample from said subject.

In yet another aspect, the invention relates to a method for predicting the risk of a cardiovascular event in a subject who has not previously shown symptoms of cardiovascular disease and/or has not previously been diagnosed with a cardiovascular disease, comprising determining in a sample from said subject the expression level of at least one gene selected from the group consisting of PPBP, GP6, PF4, GP9, PDGFC and ITGA2B.

The mRNA biomarkers referred to above include mRNA transcription products of the genes PPBP, GP6, PF4, GP9, PDGFC and ITGA2B. The protein biomarkers referred to above include translational products of these mRNA molecules.

The invention further relates to use of at least one gene selected from the group consisting of PPBP, GP6, PF4, GP9, PDGFC and ITGA2B, or of at least one expression product thereof as a biomarker for predicting the risk of a cardiovascular event or the mortality risk after a cardiovascular event in a subject suffering from end-stage renal disease.

### DETAILED DESCRIPTION

The present invention relates to a method for predicting the risk of a cardiovascular event or mortality after a cardiovascular event or cardiovascular disease risk and to a method for monitoring the impact of a treatment, cardiovascular disease progression in a subject suffering from ESRD. Therefore the expression of platelet genes is determined, e.g. at least one mRNA-biomarker and/or at least one protein-biomarker is determined. The present invention further relates to the use of at least one mRNA-biomarker or at least one protein-biomarker for predicting the risk of a cardiovascular event or the mortality risk after a cardiovascular event.

The present invention particularly allows identifying ESRD patients on chronic (intermittent) hemodialysis that have an increased risk for a cardiovascular event, e.g. acute myocardial infarction, acute coronary syndrome, or stroke. Briefly, measurement of either one or more platelet markers (mRNA or protein biomarkers) will identify patients that are at an increased cardiovascular risk, and should either be monitored carefully for cardiovascular events and/or should undergo a specific prophylaxis (e.g. reagents that prevent platelet activation).

The invention is also suitable for patients that are subjected to a primary prevention (e.g. antiplatelet therapy). In these patients, the invention can be used to monitor the efficacy of the intervention.

In the following it is sometimes also referred to "biomarker(s)" or "biomarker(s) of the invention" when one or several mRNA biomarker(s) of the present invention (e.g. mRNAs corresponding to the cDNA sequences as shown in SEQ ID NO:1, SEQ ID NO:2, SEQ ID NO:3, SEQ ID NO:4, SEQ ID NO:5, SEQ ID NO:6, SEQ ID NO:7, and SEQ ID NO:8, respectively) or one or more protein-biomarker(s) of the present invention (translational product(s) of that group of said mRNAs, which are preferably polypeptide(s) comprising any of the sequences of SEQ ID NO:9, SEQ ID NO:10, SEQ ID NO:11 SEQ ID NO:12, SEQ ID NO:13, SEQ ID NO:14, SEQ ID NO:15, SEQ ID NO:16, SEQ ID NO:17, SEQ ID NO:18, and SEQ ID NO:19, as well as fragments thereof) or both, mRNA-biomarker(s) and protein-biomarker(s), are meant.

### Sample

The method of the invention comprises determining the expression of biomarkers in a sample.

The term "sample" as used herein designates a composition which is derived from the body of a subject. Preferred samples are compositions comprising blood, plasma or serum derived from a subject. The sample may be a composition which has been processed to be in a condition suitable for the method according to the invention. The processing may include incubation, centrifugation, precipitation, concentration, filtration, dialysis, and/or dilution. The type of processing may depend on the technique which is used for determining the expression of the biomarker in the sample. For example, a blood sample may be subjected to centrifugation such that plasma is obtained. In a preferred embodiment, a blood sample is obtained from the subject. Preferably, the blood sample is a whole blood sample. Preferably, the blood sample is incubated to ensure complete lysis before RNA is extracted from the blood. The sample obtained in this way may then be analysed further.

In one embodiment, the method of the invention comprises only steps which are carried out in vitro. In that embodiment, the step of obtaining the sample from the body of the subject is not encompassed by the present invention. In another embodiment, the step of obtaining the sample from the body of the subject is encompassed by the present invention.

### Determining gene expression

In the present invention, the expression of at least one gene selected from the group consisting of the PPBP, GP6 , GP9, PDGFC, ITGA2B and PF4 in a sample is determined.

The PPBP gene is defined as follows:

| | |
|---|---|
| Official symbol | PPBP |
| Official full name | pro-platelet basic protein (chemokine (C-X-C motif) ligand 7) |
| NCBI GeneID | 5473 |
| HGNC ID | HGNC:9240 |
| Organism | Homo sapiens |
| Synonyms | PBP; TC1; TC2; TGB; LDGF; MDGF; TGB1; B-TG1; CTAP3; CXCL7; NAP-2; SCYB7; THBGB; LA-PF4; SCAR10; THBGB1; Beta-TG; CTAPIII; CTAP-III |

| | |
|---|---|
| NCBI is the abbreviation of the "National Center for Biotechnology Information", U.S. National Library of Medicine, 8600 Rockville Pike, Bethesda MD, 20894 USA. HGNC is the abbreviation of the "HUGO Gene Nomenclature Committee" (contact: EMBL Outstation - Hinxton, European Bioinformatics Institute, Wellcome Trust Genome Campus, Hinxton, Cambridge, CB10 1SD, United Kingdom) | |

The GP6 gene is defined as follows:

| | |
|---|---|
| Official symbol | GP6 |
| Official full name | glycoprotein VI (platelet) |
| NCBI GeneID | 51206 |
| HGNC ID | HGNC:14388 |
| Organism | Homo sapiens |
| Synonyms | GPIV; GPVI; BDPLT11 |

The GP9 gene is defined as follows:

| | |
|---|---|
| Official symbol | GP9 |
| Official full name | glycoprotein IX (platelet) |
| NCBI GeneID | 2815 |
| HGNC ID | HGNC:4444 |
| Organism | Homo sapiens |
| Synonyms | GPIX; CD42a |

The PDGFC gene is defined as follows:

| | |
|---|---|
| Official symbol | PDGFC |
| Official full name | platelet derived growth factor C |
| NCBI GeneID | 56034 |
| HGNC ID | HGNC:8801 |
| Organism | Homo sapiens |
| Synonyms | SCDGF; FALLOTEIN |

The ITGA2B gene is defined as follows:

| | |
|---|---|
| Official symbol | ITGA2B |
| Official full name | integrin, alpha 2b (platelet glycoprotein IIb of IIb/IIIa complex, antigen CD41) |
| NCBI GeneID | 3674 |
| HGNC ID | HGNC:6138 |
| Organism | Homo sapiens |
| Synonyms | GT; GTA; CD41; GP2B; HPA3; CD41B; GPIIb; BDPLT2 |

The PF4 gene is defined as follows:

| | |
|---|---|
| Official symbol | PF4 |
| Official full name | platelet factor 4 |
| NCBI GeneID | 5196 |
| HGNC ID | HGNC:8861 |
| Organism | Homo sapiens |
| Synonyms | PF-4; CXCL4; SCYB4 |

The expression level of a gene is typically determined by determining the amount of an expression product of said gene. The expression products are preferably selected from the group consisting of mRNA and its reverse complementary DNA, cDNA and its reverse complementary DNA, and any polypeptide(s) encoded by the gene and/or the mRNA. The mRNA sequences are characterized herein by their correspondig cDNA sequences.

The following preferred expression products are associated with the genes used in the present invention.

| **Gene** | **cDNA sequence** | **Polypeptide(s)** |
|---|---|---|
| | **(SEQ ID NO)** | **(SEQ ID NO)** |
| PPBP | 1 | 9, 10, 11, 12 |
| GP6 | 2, 3, 4 | 13, 14, 15 |
| GP9 | 5 | 16 |
| PDGFC | 6 | 17 |
| ITGA2B | 7 | 18 |
| PF4 | 8 | 19 |

Preferred translational products to be detected in accordance with this invention are the polypeptides comprising any of the sequences of SEQ ID NO:9 through 19. PBP (platelet basic protein; SEQ ID NO:9) is the mature peptide of PPBP. CTAP-III (connective tissue activating protein-3; SEQ ID NO:10), β-TG (β-thromboglobulin; SEQ ID NO:11) and NAP-2 (neutrophil-activating protein-2; SEQ ID NO:12) are truncated forms of PBP which are generated by proteolytic processing. SEQ ID NO:13-15 represent three variants of GP6. The polypeptides SEQ ID NO:16-19 are proteins encoded by GP9, PDGFC, ITGA2B and PF4, respectively. The polypeptides in the sequence listing are shown without the signal peptide.

The sequences are summarized in the following.

| **SEQ ID NO:** | **Description** | **NCBI accession No.** |
|---|---|---|
| 1 | PPBP cDNA | NM_002704.3 |
| 2 | GP6 cDNA (1). Transcript Variant: This variant (1) represents the longer transcript and encodes the longer isoform (1). | NM_001083899.1 |
| 3 | GP6 cDNA (2). Transcript Variant: This variant (2) uses an alternate splice site in the 3' coding region, compared to variant 1, that results in a frameshift. It encodes isoform 2 which has a shorter and distinct C-terminus compared to isoform 1. | NM_016363.4 |
| 4 | GP6 cDNA (3) Transcript Variant: This variant (3) lacks an exon and uses an alternate splice site in the 3' coding region, compared to variant 1, that results in a frameshift. It encodes isoform 3 which has a shorter and distinct C-terminus compared to isoform 1. | NM_001256017.1 |
| 5 | GP9 cDNA | NM_000174.3 |
| 6 | PDGFC cDNA | NM_016205.2 |
| 7 | ITGA2B cDNA | NM_000419.3 |
| 8 | PF4 cDNA | NM_002619.3 |
| 9 | PBP (platelet basic protein) | NP_002695.1 |
| 10 | CTAP-III (connective tissue activating protein-3 | NP_002695.1 |
| 11 | β-TG (β-thromboglobulin) | NP_002695.1 |
| 12 | NAP-2 (neutrophil-activating protein-2) | NP_002695.1 |
| 13 | Polypeptide encoded by SEQ ID NO:2 | NP_001077368.1 |
| 14 | Polypeptide encoded by SEQ ID NO:3 | NP_057447.4 |
| 15 | Polypeptide encoded by SEQ ID NO:4 | NP_001242946.1 |
| 16 | GP9 polypeptide | NP_000165.1 |
| 17 | PDGFC polypeptide | NP_057289.1 |
| 18 | ITGA2B polypeptide | NP_000410.2 |
| 19 | PF4 polypeptide | NP_002610.1 |

The term translational products thus means herein polypeptides translated from any of the mRNA biomarkers of the invention including products of post-translational processing of polypeptides, such as the products of post-translational proteolytic processing of the polypeptides or various post-translational modifications of said polypeptides.

In the present invention, the determination of the expression of mRNA-biomarker(s) is preferred over the determination of the expression of protein-biomarker(s). This way, it may be possible to obtain more reliable data because the determination of serum concentrations of secreted proteins, such as PF4, can be flawed by platelet activation and degranulation after sample acquisition resulting in abnormally high measured concentrations.

The step of determining the expression of mRNA or protein in the sample includes determining the presence or absence of the respective mRNA or protein. Preferably, however, the step of determining the expression of mRNA or protein includes determining the amount or concentration of mRNA or protein in the sample in a quantitative or semiquantitative manner. Most preferably, the step of determining the expression of mRNA or protein includes determining the amount or concentration of mRNA or protein in the sample in a quantitative manner.

A variety of methods can be employed to determine the amount of biomarker in the sample. The type of method to be used depends on the nature of the biomarker. The expression can be determined or measured for example, using microarray based methods, PCR methods, and/or antibody based methods, as is known to a person of skill in the art.

Typically, a microarray consists of different nucleic acid probes on a substrate, which can be a microchip, a glass slide or a microsphere-sized bead. A microchip may be constituted of polymers, plastics, resins, polysaccharides, silica, aluminosilicates, borosilicates, carbon, metals, inorganic glasses, or nitrocellulose. The microarray-based detection method typically comprises the following steps: (1) Isolating RNA from a sample and optionally converting the mRNA to cDNA, and labeling this RNA or cDNA. Methods for isolating RNA, converting it into cDNA and for labeling nucleic acids are described in manuals for micro array technology. (2) Hybridizing the nucleic acids from step 1 with probes. The nucleic acids from a sample can be labeled with a dye, such as the fluorescent dyes Cy3 (red) or Cy5 (blue). Generally, a control sample is labeled with a different dye. (3) Detecting the hybridization of the nucleic acids from the sample with the probes and determining at least qualitatively, and more particularly quantitatively, the presence or the amounts of the nucleic acid corresponding to the mRNA-biomarker(s) of the present invention and optionally additional biomarker(s) different from the biomarkers of the present invention. The difference in the amount or concentration of nucleic acid corresponding to the mRNA-biomarker(s) of the present invention between samples can be estimated based on a difference in the signal intensity. These can be measured and analyzed by appropriate software. Many variants of microarray based methods are available and known to the man skilled in the art.

Alternatively and preferably, the expression of the mRNA-biomarker(s) of the present invention may e.g. be detected in a quantitative RT-PCR approach. A quantitative reverse transcription real-time PCR approach is a particularly preferred method of the present invention.

Quantitative reverse transcription real-time PCR typically comprise(s) (1) the reverse transcription of RNA (e.g. mRNA) into DNA (e.g. cDNA) using an RNA-dependent DNA polymerase (i.e. a reverse transcriptase), (2) the amplification of the DNA produced by reverse transcription using PCR, and (3) the detection and quantification of the amplification products in real time.

Amplified products produced by PCR may be detected by any of the methods known in the art. The detection of the amplification product may be conducted using labelled oligonucleotide primers or probes, e.g. labelled with a fluorescent dye. The dye may, e.g. be selected from the group consisting of LightCycler-probes (Roche), Taqman probes (Roche), FRET probes, UPL probes, molecular beacons, Scorpion-primers, Sunrise-primers, LUX-primers or Amplifluor-primers. Furthermore, non-fluorescent Quenchers may be used.

Conditions for quantitative RT-PCR or quantitative reverse transcription real-time PCR are known by the man skilled in the art, i.e. the man skilled in the art knows how to design gene specific oligonucleotide primers and probes, how to choose the number of PCR-cycles as well as suited temperatures, buffer condition and time spans for reverse transcription, denaturing steps, primer annealing steps and elongation steps. The man skilled in the art is also aware of devices and software for amplification, detection, quantification and evaluation, e.g. LightCycler480 (Roche).

When expression of a protein-biomarker is determined the expression is preferably determined by use of an antibody directed against the protein-biomarker. The antibody according to the invention may be a monoclonal antibody or an antibody derived from or comprised in a polyclonal antiserum, monoclonal antibodies are preferred. The term "antibody", as used herein, further comprises derivatives such as Fab, F(ab')2, Fv or scFv fragments: see, for example Harlow and Lane, "Antibodies, A Laboratory Manual" CSH Press 1988, Cold Spring Harbor N. Y. Fab, F(ab')2 and Fv fragments fragments can be obtained by digesting a complete antibody with papain, pepsin, etc. in the conventional manner. The antibody or the derivative thereof may be of natural origin or may be (semi)synthetically produced. Such synthetic products also comprise non- proteinaceous or semi-proteinaceous material that has the same or essentially the same binding specificity as the antibody of natural origin. Such products may, for example be obtained by peptidomimetics. The antibody may be used in methods which are known to those skilled in the art, e.g. ELISA or flow cytometry.

In the ELISA method, an antibody, preferably a monoclonal antibody, or a fragment thereof against a protein-biomarker of the present invention is usually first immobilized on a carrier as a primary antibody. Preferred carrier is a solid carrier, such as an ELISA plate container molded from a carrier polymer such as styrene or polystyrene. The monoclonal antibody or its fragment can be immobilized by, for example, dissolving the monoclonal antibody or its fragment in a buffer such as a carbonate buffer or a borate buffer followed by the adsorption onto the carrier.

Separately, an antibody (a monoclonal antibody or a polyclonal antibody, or a fragment thereof) used as the secondary antibody is labeled preferably with a non-radioactive label. Enzyme labels, fluorescent labels, light emission labels, etc. can be used as the non-radioactive label. It is preferred that an enzyme label such as alkaline phosphatase, β-galactosidase or horse radish peroxidase be used.

In one aspect of the invention, the expression of one or more biomarkers of the present invention is determined in at least two samples, or at least 3 samples, or at least 4 samples, or at least 5 samples obtained from the same subject suffering from ESRD at different points of time. This may include collecting data over a period of time. Samples from the subject may be taken at regular intervals. The intervals range e.g. from about 2 months to about 12 months, preferably it ranges from about 4 months to about 6 months. This allows the monitoring of the impact of a treatment administered to the subject on the risk of a cardiovascular event or the monitoring of the progression of a cardiovascular disease in the subject.

### Subject

A subject suffering from end-stage renal disease (ESRD) frequently suffers also from accelerated vascular disease. The subjects suffering from ESRD in the present invention include those subjects who have or have not previously suffered symptoms of and/or have or have not been diagnosed with a cardiovascular disease. Preferably, the subject suffering from ESRD has not previously suffered symptoms of or has not been diagnosed with a cardiovascular disease.

A subject suffering from ESRD is defined herein as a subject undergoing renal replacement therapy. In the present invention, the subject suffering from ESRD is preferably a subject who undergoes hemodialysis (chronic (intermittent) hemodialysis). When referring to a subject suffering from ESRD in the following it is meant to include any of the above identified subjects.

Usually, the subject in the present invention is human.

### Cardiovascular disease, risk prediction and monitoring

The method of the present invention is useful for risk stratifying a subject for a future cardiovascular event, wherein the subject suffers from ESRD and has or has not previously suffered symptoms of or been diagnosed with a cardiovascular disease. It may be particularly useful for risk stratifying a subject for a cardiovascular event who has not previously suffered symptoms of or been diagnosed with a cardiovascular disease.

In the present invention, cardiovascular events include, but are not limited to, (1) acute myocardial infarction, (2) stroke, (3) acute coronary syndrome, (4) sudden death related to cardiac arrhythmias, and (5) amputation and/or invasive intervention(s) related to peripheral vascular disease.

In another embodiment, the present invention relates to a method for predicting the mortality risk after a cardiovascular event in a subject suffering from end-stage renal disease, comprising determining the expression of at least one mRNA-biomarker selected from the group of mRNAs represented by the cDNA sequences SEQ ID NO:1, SEQ ID NO:2, SEQ ID NO:3, SEQ ID NO:4, SEQ ID NO:5, SEQ ID NO:6, SEQ ID NO:7, and SEQ ID NO:8, or at least one protein-biomarker selected from the group of translational products of said mRNAs in a sample from said subject.

In one embodiment, the method of the present invention relates to a method for predicting the risk of a cardiovascular event or mortality after a cardiovascular event over a period of two years.

In yet a further embodiment, the present invention relates to a method for monitoring the impact of a treatment administered to a subject suffering from end-stage renal disease on the risk of a cardiovascular event, comprising determining the expression of at least one mRNA-biomarker selected from the group of mRNAs represented by the cDNA sequences SEQ ID NO:1, SEQ ID NO:2, SEQ ID NO:3, SEQ ID NO:4, SEQ ID NO:5, SEQ ID NO:6, SEQ ID NO:7, and SEQ ID NO:8, or at least one protein-biomarker selected from the group of translational products of said mRNAs in a sample from said subject. In this context renal replacement therapy such as hemodialysis is considered a "treatment". However, the term "treatment" here preferably refers to the administration of a pharmaceutically active ingredient, more preferably a pharmaceutically active ingredient for treating a cardiovascular disease, to the subject suffering from ESRD. In one embodiment the term treatment e.g. relates to pharmaceutically active ingredient(s) that prevent and/or or decrease platelet activation. In a patient subjected to a primary prevention treatment, e.g. antiplatelet therapy, the biomarkers of the present invention can thus be used to monitor the impact, i.e. the efficacy of the treatment.

In another embodiment, the present invention relates to a method for predicting a cardiovascular disease or for monitoring the progression of a cardiovascular disease in a subject suffering from end-stage renal disease, comprising determining the expression of at least one mRNA-biomarker selected from the group of mRNAs consisting of SEQ ID NO:1, SEQ ID NO:2, SEQ ID NO:3, SEQ ID NO:4, SEQ ID NO:5, SEQ ID NO:6, SEQ ID NO:7, and SEQ ID NO:8, or at least one protein-biomarker selected from the group of translational products of said mRNAs in a sample from said subject.

The cardiovascular disease includes:
● coronary heart disease - disease of the blood vessels supplying the heart muscle
● ischemic cerebrovascular disease - disease of blood vessels supplying the nervous system
● peripheral vascular disease - disease of blood vessels supplying the extremities

Accordingly, the subject who has previously been diagnosed with a cardiovascular disease or whose progression of the cardiovascular disease is monitored or for whom a cardiovascular disease is predicted in the different embodiments of the invention may have suffered/will suffer from e.g. myocardial infarction, stable or unstable or effort angina pectoris, stroke and/or peripheral vascular disease. A subject who suffered symptoms of a cardiovascular disease in the meaning of the present invention includes a subject that only suffered symptoms of a cardiovascular disease without actually suffering from a cardiovascular disease. For example, a subject who suffered symptoms of a cardiovascular disease is a subject who had suffered e.g. acute symptoms, such as chest pain, that could originate from a cardiovascular disease, e.g. acute myocardial infarction, prior to sample taking. The symptoms could however also relate to a different disease, such as e.g. gastro-esophageal reflux disease in the case of a chest pain patient.

As the biomarkers of the present invention have prognostic value the method according to the invention also relates to a method wherein the subject has no symptoms of a cardiovascular disease or no acute symptoms of a cardiovascular event. The present invention also relates to a method, wherein the subject has e.g. no symptoms of a cardiodvascular event, such as e.g. chest pain or wherein the subject has not had any symptoms of a cardiodvascular event at the time of sample taking and/or e.g. within at least 2 months or at least 2 weeks, or within 72 hours or 48 h or 24 prior to sample taking.

In one embodiment of the present invention, the method comprises comparing the expression of a biomarker of the present invention with a predetermined value. As used herein, the term "predetermined value" refers to the amount or concentration of the biomarker of the present invention in a sample, obtained from the general population or from a selected population of subjects. A selected population of subjects may e.g. be subjects suffering from ESRD who died from a cardiovascular disease or after having suffered a cardiovascular event. The differential between the predetermined value and the amount or concentration of the biomarker in the sample of a subject may then be indicative for the risk of a cardiovascular event, the morbidity risk after a cardiovascular event, the prediction of or the progression of a cardiovascular disease in that subject.
In one embodiment, the inventions thus also relates to a method as described herein, wherein the expression of the at least one mRNA-biomarker or at least one protein-biomarker in the sample from the subject suffering from end-stage renal disease is compared with the expression of said mRNA biomarker or protein biomarker in one or more samples which were obtained from one or more other subjects suffering from end-stage renal disease having suffered a cardiovascular event (e.g. within two years after sample taking and not having survived for longer than two years since sample taking), wherein a decreased expression predicts a decreased risk of a cardiovascular event or mortality risk after a cardiovascular event.

In the method of the present invention the determination of the expression of ITGA2B-mRNA, PPBP-mRNA or PF4 mRNA are preferred.

It is furthermore preferable in the present invention to determine the expression of 2 or more, more preferable of 3 or more, further more preferable of 4 or more, and particularly preferable of 5 or more of the mRNA-biomarkers of the present invention.

The method of the present invention may preferably also comprise the determination of additional parameters besides the determination of the biomarker(s) of the present invention. Inclusion of additional parameters into the risk prediction of a cardiovascular event or mortality after a cardiovascular event or for predicting a cardioavascular disease in the present invention may increase the predictive power. Additional parameters in the present invention include clinical parameters, e.g. age and the previous history of cardiovascular disease. The term "determination of additional parameters" may further comprise the determination of other biomarkers different from the biomarkers of the present invention.

As another embodiment, the present invention is directed to the use of at least one mRNA selected from the group of mRNAs represented by the cDNA sequences SEQ ID NO:1, SEQ ID NO:2, SEQ ID NO:3, SEQ ID NO:4, SEQ ID NO:5, SEQ ID NO:6, SEQ ID NO:7, and SEQ ID NO:8, or of at least one translational product selected from the group of translational products of said mRNAs as a biomarker for predicting the risk of a cardiovascular event or a mortality risk after a cardiovascular event.

The preferred embodiments of one aspect of the invention apply to the other aspects of the invention *mutatis mutandis.*

The example hereafter is intended to illustrate the invention without however limiting it.

### Example

### BRIEF DESCRIPTION OF THE TABLES AND DRAWINGS

### Table 1:

(A) Clinical data of 321 ESRD patients on chronic intermittent hemodialysis.
(B) Clinical data, comparing the low- and high-risk ESRD patients.

### Table 2: Identification of genes with a non-zero effect on cardiovascular events

Non-zero regression coefficient estimates from the subdistribution hazard model are presented in the second column. The model "death from other cause" is presented in the third column ('Other Death').

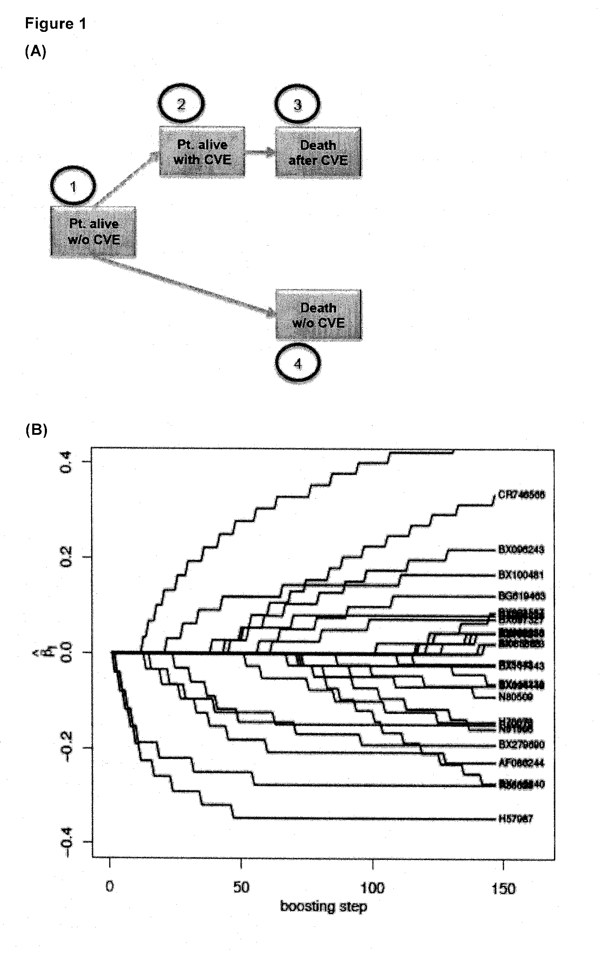

### Figure 1: A risk prediction model that links high-dimensional molecular data to complex clinical endpoints

(A) Potential states of a patient: The transition indicated by the dashed arrow and therefore the exact timing of the state "alive with cardiovascular event (CVE)" cannot be observed exactly. Therefore, the genes are linked to the transition for state [1] to state [3] and the transition from state [1] to state [4]. The difference between these two types of transitions can be attributed to the dashed components, i.e., to the occurrence of cardiovascular events.
(B) Illustration of component-wise boosting model building steps for obtaining sparse risk prediction models, i.e., signatures comprising only few genes with an effect on the transition, for the two types of transitions. Model building starts at the left side with all effects equal to zero. In each step only one effect estimate is updated, while the others are kept fixed. The number of steps is determined by cross-validation, optimizing prediction performance for new patients.

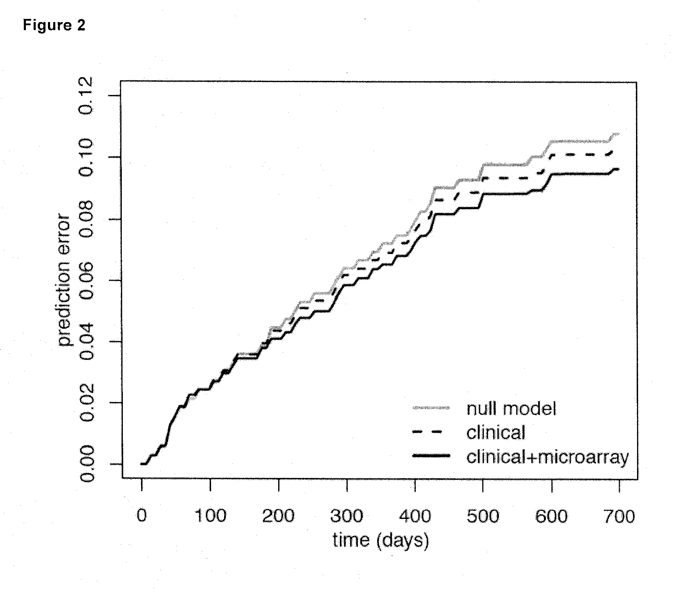

### Figure 2: Improved prediction of cardiovascular events, combining clinical and microarray data

Shown are the prediction error curves for the cumulative incidence of death after a cardiovascular event in the course of time, estimated by the bootstrap .632+ approach. The subdistribution hazard model, incorporating both clinical covariates and gene expression data *('clinical + microarray',* solid black curve) is superior to either a model without covariates *('null model',* grey line) or a model based on clinical risk factors alonel *('clinical',* dashed black line). This analysis demonstrates that the microarray data has predictive value beyond the clinical covariates. The added value is the difference between the solid and the dashed black curve.

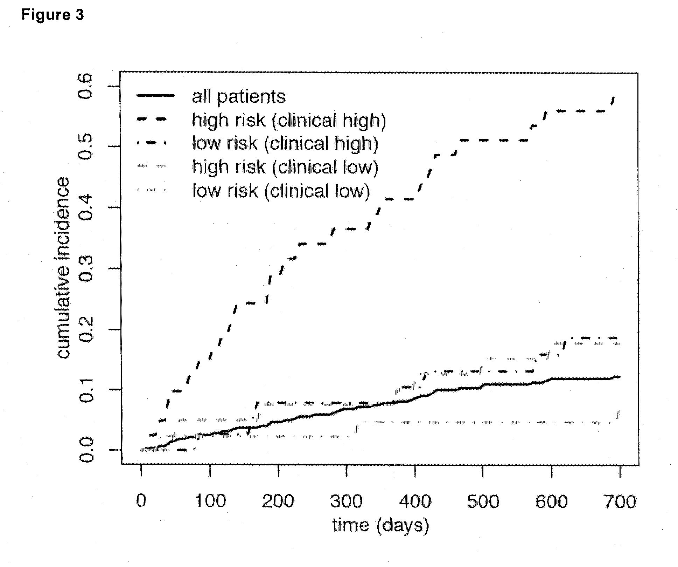

### Figure 3: The integration of gene expression data separates high- from low-risk patients with respect to death after a cardiovascular event

The solid line *(all patients)* depicts the cumulative incidence of death after a cardiovascular event in the course of time for all patients. The cumulative incidence was then calculated for high- and low-risk groups, based on clinical risk factors ("clinical high" and "clinical low"), and combined with the risk prediction signature ("high risk" and "low risk"). The dashed black line depicts the cumulative incidence of death in patients with a high clinical risk and a high-risk gene profile *(high risk (clinical high)),* the grey dash-dotted line depicts the cumulative incidence of death in patients with a low clinical risk and a low-risk gene profile *(low risk (clinical low)),* the black dash-dotted line depicts the cumulative incidence of death in patients with a high clinical risk and a low-risk gene profile *(low risk (clinical high)),* and the grey dashed line depicts the cumulative incidence of death in patients with a low clinical risk and a high-risk gene profile *(high risk (clinical low)).*

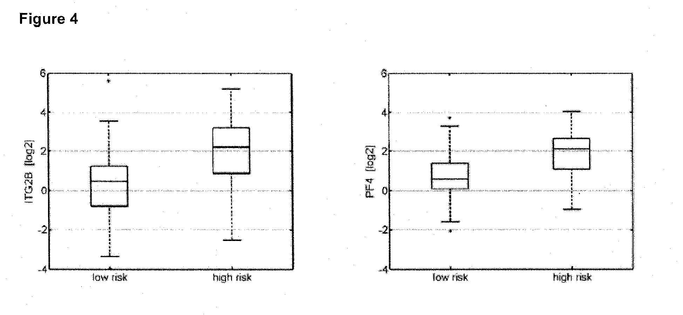

### Figure 4: Increased expression of ITGA2 and PF4 in patients with high cardiovascular risk

Distribution of the gene expression data obtained by quantitative RT-PCR (RT-qPCR) in patients with low cardiovascular risk (n= 81) versus patients with high cardiovascular risk (n= 81), classified by the subdistribution hazard model. RT-qPCR revealed a 3.24-fold increase of ITGA2B (ANOVA; p= 1.92•10⁻⁸), and a 2.33-fold increase of PF4 (ANOVA; p= 4.81•10⁻⁹) in the high-risk group compared to the low risk group.

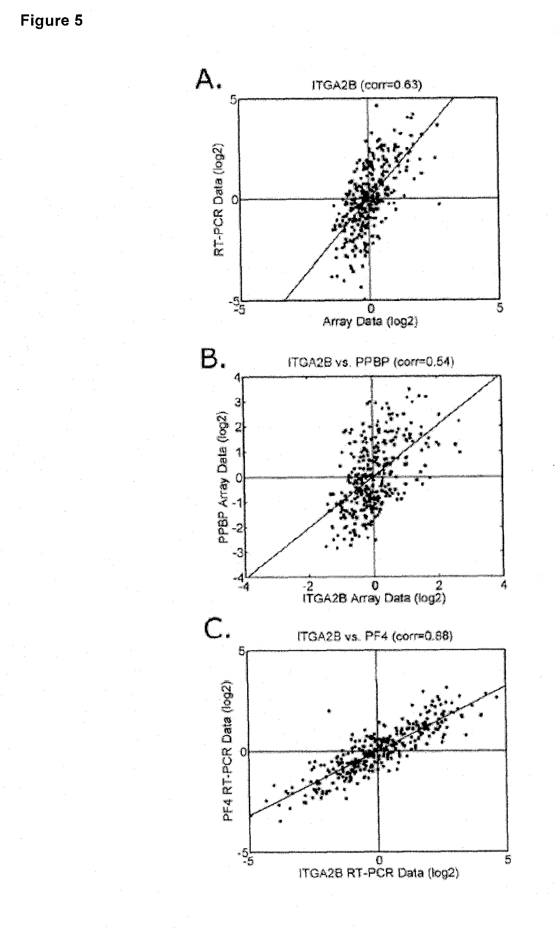

### Figure 5: Correlations between the ITGA2 and PPBP microarray data and the RT-PCR results

(A) The correlation between the RT-qPCR and microarray data demonstrates that ITGA2 is regulated across the patients because only a regulated gene can generate correlated measurements. (B) The platelet protein PPBP is regulated in a correlated fashion with ITGA2 as indicated by the microarray measurements. (C) The RT-qPCR measurements of PF4 are strongly correlated with the expression of ITGA2 (corr = 0.88).

### Methods

### Study Design and Population

This study was designed to identify a potential link between the gene profiles of circulating blood cells of hemodialysis patients and the occurrence of cardiovascular events over a 2-year observation period, wherein a cardiovascular event included acute myocardial infarction, stroke, sudden death related to cardiac arrhythmias, and amputation / invasive interventions related to peripheral vascular disease. The institutional ethics committee at the University Hospital Freiburg approved the protocol; the study was conducted in accordance with the Declaration of Helsinki at four outpatient hemodialysis centers in Germany. After obtaining informed consent, blood samples were collected from 324 hemodialysis patients immediately before dialysis treatment following a two-day dialysis-free interval; 3 samples were excluded due to poor RNA quality, the remaining 321 samples were processed as outlined below. Nineteen clinical covariates, including age, sex, duration of dialysis, previous cardiovascular events, were recorded at the time of enrollment; clinical chemistry, including lipid profile and hematological parameters, were extracted from the patients' records. Patients were subsequently followed for two years. As we could not directly observe the time of cardiovascular events, patients were monitored for two other types of events that allow for an indirect link of gene profiles to cardiovascular events: We monitored for death, an used patient records for identifying whether a patient had a cardiovascular event prior to death (without requiring a casual link). Thus, we effectively monitored patients for "death with prior cardiovascular event" and "death without prior cardiovascular event".

### RNA preparation

Whole blood specimens were collected in 2 x 2.5 ml PAXgene™ tubes from each subject, incubated at room temperature for 3 h to ensure complete lysis, and then stored at -80 °C. RNA was extracted from whole blood using the PAXgene™ Blood RNA System (PreAnalytiX GmbH, Belgium), following the manufacturer's instructions. The quality of the purified RNA was verified on an Agilent® 2100 Bioanalyzer (Agilent Technologies, Palo Alto, CA). RNA concentrations were determined using a GeneQuant II RNA / DNA Calculator (Pharmacia).

### Microarray Processing

Each RNA sample was amplified using the MessageAmp II aRNA kit (Ambion, Austin TX), using 1μg of total RNA according to the manufacturer's instructions. In addition, Universal Human Reference RNA (Stratagene) was amplified and pooled as a reference for all hybridizations. cDNA microarrays were produced and processed essentially according to the Stanford protocol described by Eisen and Brown (26). Approximately 38,000 annotated genes (Human Unigene Set - RZPD 3.1) from the RZPD (Resource Center and Primary Database, Berlin, Germany) were obtained as bacterial stocks, and amplified by polymerase chain reaction (PCR). PCR products were purified and transferred into 384-well plates. Printing was performed on aminosilane-coated slides (CMT-GAP II Slides, Corning, NY), using a Qarray2 arrayer from Genetix Ltd. (http://www.genetix.com/en/home/index.html). Post-processing was performed according to the Stanford protocol (http://brownlab.stanford.edu/). Hybridizations were performed in the presence of an equal amount of amplified reference RNA (Stratagene, La Jolla, CA, USA) as published (13). All other steps, including hybridization, were performed following the Brown protocol. Signal intensities of the hybridized slides were quantified using an Axon 4000A scanner in combination with the GenePix Pro 6.0.1.17 image processing software (Axon Instruments, Union City, USA). After background subtraction, the log₂ of the medians of the pixel intensity ratios Cy5/Cy3 within a spot were used to measure the expression of a gene relative to its abundance in the Universal Human Reference RNA. The Locally Weighted Scatterplot Smoothing (LOWESS) algorithm with a smoothing parameter of 0.2 was applied to adjust for the intensity-dependent bias between the two fluorophores (19). Print-tip effects were corrected by a block-wise normalization procedure (59). The Genedata Expressionist Refiner Pro 4.0 software (Genedata AG, Basel, Switzerland) was used for quality control.

### Quantitative RT-PCR

Quantitative RT-PCR (quantitative reverse transcription real-time PCR) experiments were performed using the Roche real-time PCR master mix (Lightcycler 480 Probes Master) in combination with the Roche Universal Probe Library (UPL) assays. All measurements were performed using the Roche Light Cycler 480 (Roche). Ribosomal protein L32 (RPL32) was used as a housekeeping gene (5'ccaccgtcccttctctctt3'; 5'gggcttcacaaggggtct3') to quantify the expression of ITGA2B (5'gagacacccatgtgcagga3'; 5'agctggggcacacatacg3'), and PF4 (5'agcctggaggtgatcaagg3'; 5'gaagaccacctcccaggtc3'); all measurements were carried out in triplicates. Negative controls included a sample without reverse transcriptase and a sample without template. The regulation of genes was calculated using the normalized data derived from the relative quantification analysis.

### Data Analysis

The data was analyzed by considering the instantaneous risk, i.e., the hazard, for cardiovascular events in Cox proportional hazard regression models. However, the time of cardiovascular events could not be observed exactly. Therefore, an indirect analysis was performed with Cox models for the events "death with prior cardiovascular event" and "death without prior cardiovascular event" (Figure 1A). We differentiated between the states [1] "patient alive without cardiovascular event", [2] "patient alive with cardiovascular event", [3] "death after cardiovascular event" and [4] "death without prior cardiovascular event". While the exact time points for reaching state [3] or [4] can be recorded, this is not the case for the transition between [1] and [2]. However, each patient entering state [3] has to pass through state [2]. Therefore, the cumulative incidence of [3], i.e. the proportion of observed deaths after a cardiovascular event in the course of time, is the cumulative incidence for [2] multiplied by the probability of dying of any cause. If a gene is linked to [3], this could either be due to a linkage to [2], or due to a linkage to death in general. To distinguish between these two possibilities, we considered two types of statistical models. The first model investigated the connection between gene expression and the cumulative incidence for state [3]. The second model linked gene expression to the risk for reaching state [4]. If a gene had an effect only in the first type of model, but not in the second, it had to be linked to the transition from state [1] to state [2], but not to the transition between [2] and [3]. Therefore, the gene had to be linked to cardiovascular events.
To identify differentially regulated genes linked to future cardiovascular events, all genes were considered simultaneously in a multivariable model that takes correlations between genes into account. A component-wise boosting approach for the Cox regression model (11) (Figure 1B) was implemented to identify a small number of important genes with a predictive value beyond the information contained in clinical covariates. Specifically, age and prior cardiovascular events were included as mandatory covariates to compare the prediction performance of this approach with a purely clinical model that incorporated only clinical covariates. We utilized the subdistribution hazard approach of Fine and Gray (30) to extend the Cox regression, and to directly link gene expression to cumulative incidence, i.e., the observed proportion of deaths after cardiovascular events over time. Using the componentwise boosting approach, the subdistribution hazard approach was adapted for a highdimensional setting (9), yielding a list of genes with non-zero effects and corresponding regression parameters that specified the direction and size of the effect. The estimated regression parameters for a model for "death from other cause", i.e. moving from state [1] to [4], were also obtained by component-wise boosting (11). For comparison, a subdistribution hazard model was developed that contains only clinical covariates, where age and prior cardiovascular events were again included as mandatory variables. Since the number of steps of the boosting algorithm determines the complexity of all models, it was selected by 10-fold cross-validation to avoid over-fitting. Although prediction performance of the fitted subdistribution hazard model is usually evaluated on a separate set of patients, this reduces the number of observations available for model fitting, decreasing the power to identify important genes. Therefore, we used all available patient data to fit the model. To evaluate the prediction performance for new patients, we employed a bootstrap technique. Briefly, 100 bootstrap data sets were generated, each containing a random sample of 0.632n patients of the original data (10). Model development for the subdistribution hazard model was performed for each bootstrap data set, including 10-fold cross-validation. The prediction performance was evaluated by "bootstrap 0.632+" estimates; the latter provides reliable estimates of prediction performance for new patients in a time-to-event setting (10, 33). The Brier score, i.e. the squared difference between the predicted probability that a cardiovascular event will occur and the true status, was calculated and tracked over time, resulting in prediction error-curve estimates (9, 50). The Aalen-Johansen estimator of the cumulative incidence, a performance reference that does not employ covariate information, was used as a null model, i.e., only the observed proportion of cardiovascular events at each time point is used to predict the risk for a new patient in the course of time, regardless of the patient characteristics. Model fitting as well as estimation of prediction performance was performed in the statistical environment R.
The risk prediction model for "death with prior cardiovascular event" provided a small set of genes with good prediction performance. However, due to the limited number of events and the use of cross-validation, not all relevant genes are contained in this risk prediction signature. Therefore, we chose a complementary approach to extend this risk prediction signature. Using the risk prediction signature, each patient was assigned a predicted risk, yielding a group with high risk and a group with low risk, corresponding to the upper and the lower quartiles of the predicted risk. The two groups consisted of 81 patients with a predicted high risk, and 81 patients with a predicted low risk for cardiovascular events. Subsequently, we searched for differentially expressed genes between these two groups, using a two sample t-test. The false discovery rate (FDR) was set at pFDR <0.05 to control for multiple testing (7), using the Genedata Analyst software (Genedata AG, Basel, Switzerland). Differentially expressed genes were then analyzed by the Gene Set Analysis (GSA) (25), and confirmed by RT-PCR measurements using analysis of variance (ANOVA).

### Results

### Patient survival

Informed consent and blood samples were obtained from 324 patients; three samples were discarded due to insufficient RNA quality. For the remaining 321 patients (Table 1A), all relevant data were collected over a 2-year observation period. The mean age was 66 years (61 % male; 39% female). As predicted from other studies, the one-year mortality in this population exceeded 15%, and 87 (27%) of the 321 patients enrolled into this study died during the two-year observation period.

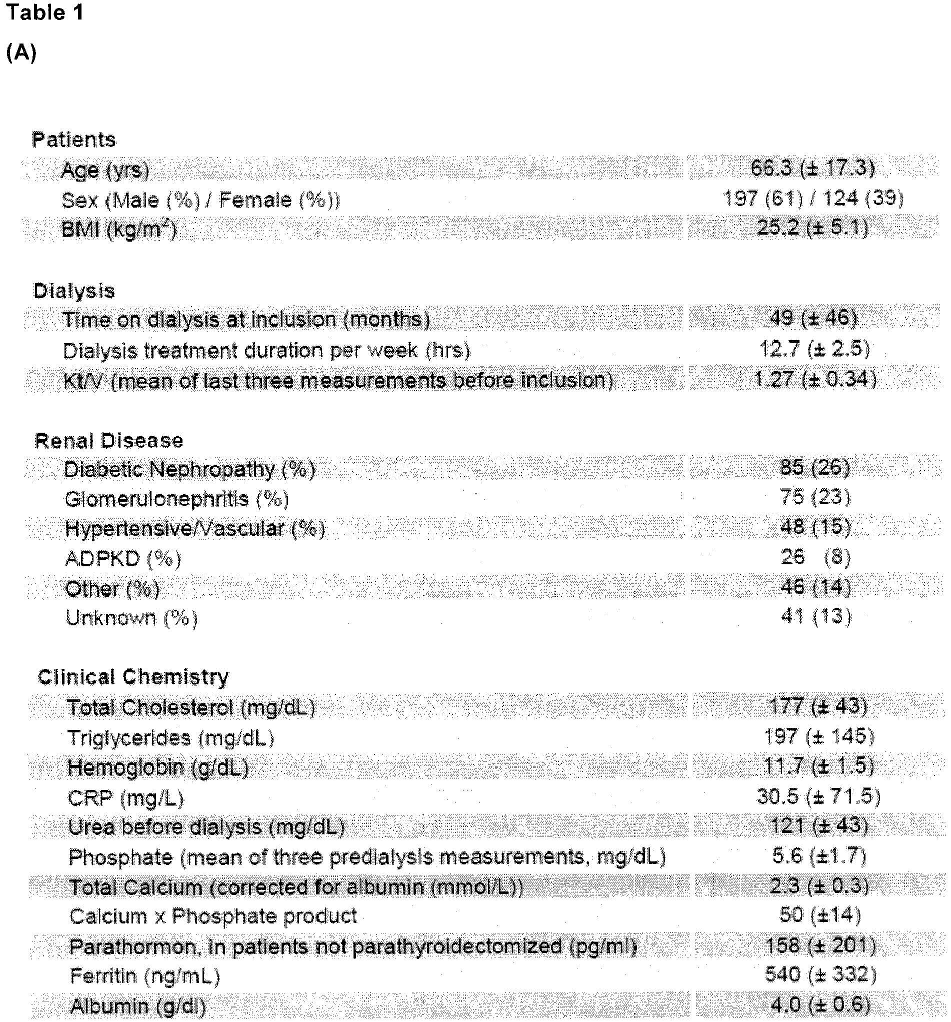

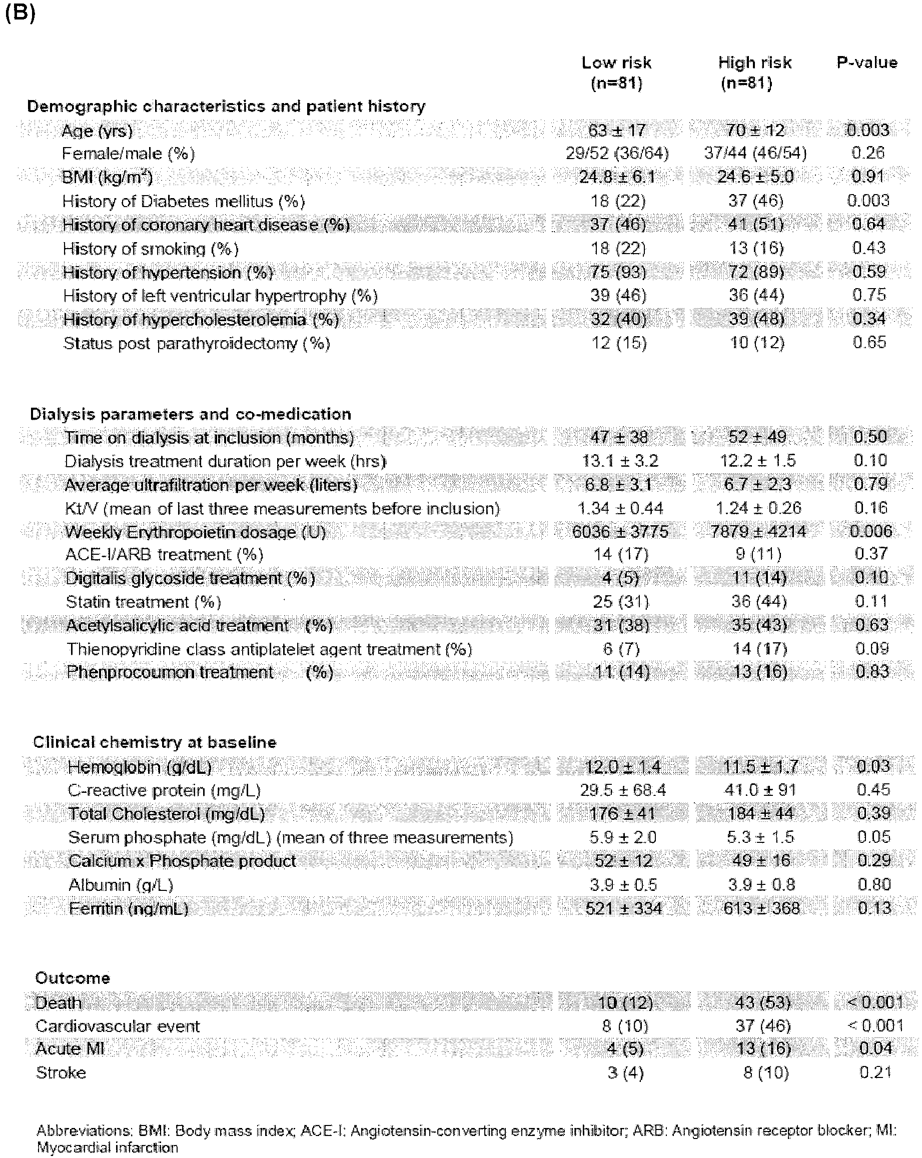

### A subdistribution hazard model for predicting the risk for cardiovascular events

To identify genes that predict cardiovascular events at the beginning of a two-year observation period, we developed a subdistribution hazard model that extracted information from the microarray data beyond the risk prediction defined by clinical parameters such as age and the presence of coronary artery disease at the beginning of the observation period. Specifically, we developed a model that took the effect of all clinical covariates into account and included gene expression predictors only if these showed prediction performance in addition to the clinical covariates. The model was based on the data of 321 patients with 39 cardiovascular events and death during the follow-up period, 48 deaths without prior cardiovascular event, and 234 patients that were alive, or transplanted during the two-year observation period. The component-wise boosting used to fit the subdistribution hazard model, resulted in estimated regression parameters for most genes that were equal to zero, i.e. these genes had no or no relevant predictive value. Only 27 genes received non-zero estimated regression parameters, thus predicting an increased risk for cardiovascular events. These 27 gene expression covariates were selected automatically by a statistical procedure to maximally improve prediction performance beyond the information from the clinical covariates. Since the gene expression data were standardized before model building, genes with larger regression parameters had a stronger effect. A positive value indicated an increasing probability of experiencing a cardiovascular event, while negative values indicated a decreased risk. Estimated regression parameters for the genes with nonzero effect were presented as log relative risks (Table 2). There was no overlap between the genes identified by the subdistribution hazard model, linking genes to "death without prior cardiovascular event" (state [4]) (Table 2), suggesting that all genes with non-zero coefficients are directly related to the occurrence of cardiovascular events. Models utilizing clinical data were better than the null model not using any covariate information (Figure 2). However, the subdistribution hazard model combining clinical and microarrays performed superior to than the clinical model alone, supporting the hypothesis that the gene expression data contained predictive value beyond the clinical covariates (Figure 2). This analysis *inter alia* identified Caspase 12 (Clone: BX096243), Mucin 20 (Clone: BX115840), and JAK2 (Clone: BX097327), genes previously implicated in renal disease (15, 36, 44) or cardiac abnormalities (6, 20, 47, 54, 58).

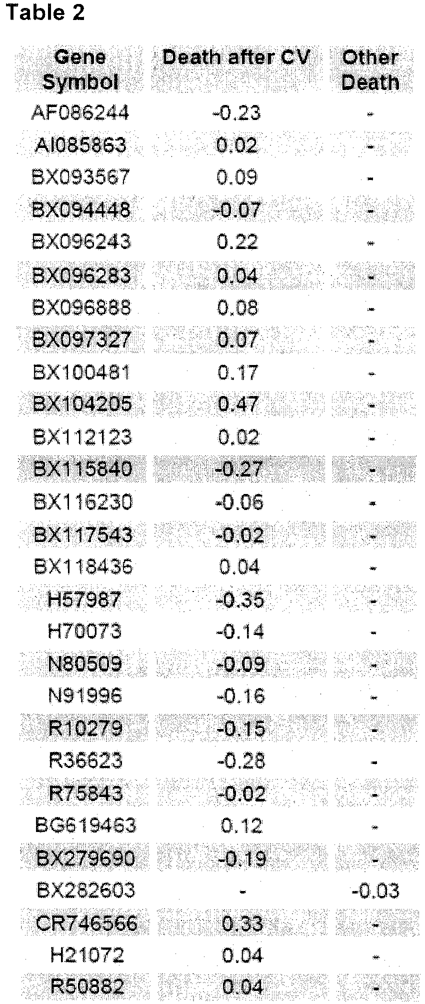

To test the value of the risk prediction signature, the cumulative incidence of deaths after a cardiovascular event was calculated for patients with a high and low clinical risk for a cardiovascular event (Figure 3). To obtain a high-risk and a low-risk group based on clinical data ("clinical high" and "clinical low"), patients were split at the median based on clinical variables. For the gene expression part, the median was used to separate patients into a "high-risk" and "low-risk" gene profile group. While the gene expression-based grouping provided only little additional separation in the clinical low-risk group (Figure 3, grey curves), considerable separation was seen for the clinical high-risk group (Figure 3, black curves). Of note, the group with a high clinical risk but a low-risk gene expression profile (dash-dotted black curve) remained close to the clinical low-risk groups, suggesting that the gene expression data can identify low-risk patients within a group of patients considered at a high risk for cardiovascular events based on their clinical risk factors. In patients with a high clinical risk ("clinical high") and an unfavorable gene profile ("high risk"), death after a cardiovascular event was observed in approximately 60% of the patients over the course of two years, revealing the strength of the prediction model.

### Transcriptional profile of hemodialysis patients with increased cardiovascular risk

Based on the results of the subdistribution hazard model and the risk prediction signature, the 321 patient samples were divided into a group of 81 samples with low cardiovascular risk, and 81 samples with high cardiovascular risk (Table 1 B), corresponding to the lower and the upper quartile of the predicted risk. As expected, patients in the high-risk group, based on their genetic profile, were significantly older, and had significantly more often a history of diabetes mellitus. Serum phosphate and hemoglobin were lower in the high-risk group, while the weekly erythropoietin dosage was increased in comparison to the low-risk group. Overall death, cardiovascular events and acute myocardial infarctions were also more prevalent in the high-risk group, while all other parameters did not differ significantly between these two groups (Table 1 B). The comparison between low- and high-risk samples identified 360 genes with a >1.5-fold up-regulation, and 105 genes with a >1.5 fold down-regulation. The upregulation was significant (p<0.05) for 340 genes, and the down-regulation for 96 genes ; correction for multiple comparisons (pFDR <0.05) removed only a few genes. Thus, the risk prediction signature encompassing 27 genes allowed us to identify additional genes that were differentially regulated between patients with low and high cardiovascular risk.

### Increased expression of platelet makers in ESRD patients with high cardiovascular risk

Up-regulation of platelet-derived proteins, including the pro-platelet basic protein (PPBP), the precursor for Platelet Basic Protein (PBP) and other truncated molecules such as NAP-2, CTAP-III, β-Thromboglobulin (14) (1.9-fold, p=2.76·10⁻⁵), Glycoprotein IX (GP9) (1.36-fold, p= 5.44·10⁻⁶), Glycoprotein VI (GP6) (1.26-fold, p= 3.64·10⁻⁵) and Platelet derived growth factor C (PDGFC) (1.22-fold, p= 0.0008) suggested that an increased baseline activation of platelets exposes ESRD patients to a higher cardiovascular risk. Furthermore, ITGA2B (BX281671), a component of the platelet glycoprotein (GP) IIb/IIIa complex that mediates adhesion to extracellular matrix proteins and activation of platelets, showed a 1.46-fold up-regulation (p= 8.47·10⁻⁶) in the high versus the low risk group. While ITGA2B (integrin alpha 2b; CD41) mutations cause Glanzmann thrombasthenia (21), activation of the glycoproteins IIb/IIIa complex triggered by conformational changes plays a central role in thrombus formation and acute myocardial infarction (4). Quantitative RT-PCR (RT-qPCR) was performed to validate the microarray data. Using RPL32 as a housekeeping gene (42), RT-qPCR revealed a 3.24-fold increase of ITGA2B in the high-risk group compared to the low-risk group (ANOVA; p= 1.92·10-⁸) (Figure 4). RT-qPCR revealed that Platelet Factor 4 (PF4), a platelet chemokine not represented on our microarray, was up-regulated 2.33-fold in the high-risk group compared to the low risk group (ANOVA; p= 4.81·10⁻⁹) (Figure 4). ITGA2B and PF4 were closely correlated to each other as well as to PPBP (Figure 5).

### Discussion

The high cardiovascular morbidity and mortality in patients with ESRD and chronic maintenance-hemodialysis remains an unsolved problem. The grim prognosis of ESRD, particular during the first year, has not significantly changed over the past years (22); despite significant technological advances and use of extensive resources, dialysis patients suffer a >20% annual mortality rate in most countries (34). It is remarkable that even young patients, typically not at risk for cardiovascular disease, face a dramatic increase in mortality once exposed to hemodialysis (23). Another concerning aspect is the complete failure of lipidlowering drugs, typically powerful agents to combat cardiovascular disease, to improve the survival of hemodialysis patients (27, 56). Moreover, anticoagulation and platelet inhibitors negatively affect survival of hemodialysis patients; in a large meta-analysis, patients exposed to warfarin, clopidogrel, and/or aspirin had significantly increased mortality rates (18, 17). These observations suggest that the prevalence of cardiovascular disease is only partly explained by conventional risk factors, and that the mechanisms causing cardiovascular disease and death in hemodialysis patients differ from other populations (31).

### The prediction of cardiovascular events in patients with ESRD

We applied an unbiased approach to identify targets to predict or treat cardiovascular disease in hemodialysis. We assumed that the gene profile of peripheral blood cells might mirror some of the pathological changes that lead to cardiovascular disease in this population, and determined the gene expression patterns of 321 hemodialysis patients prior to their hemodialysis treatment after a two-day dialysis-free interval. We followed all patients for subsequent events over a period of two years, and analyzed each cause of death. As predicted by similar observational studies, there was a significant two-year mortality; 87 of 321 patients died during the course of two years. Since the onset of cardiovascular disease cannot not be precisely recorded, we developed a novel strategy for linking high-throughput data to the risk for cardiovascular events. First, a risk prediction model, incorporating a large number of gene expression measurements, was combined with classical clinical predictors to identify prognostic genes. Most analyzed genes did not contribute towards improving risk prediction beyond clinical risk factors; however, a small set of genes improved the risk prediction for new patients, indicating that the microarray data contained risk-relevant information. Based on this risk prediction signature, a subset of patients with high cardiovascular risk was defined in a second step, and compared to a group of patients with low cardiovascular risk. This two-step analysis yielded multiple differentially regulated genes.

### Platelet activation as a predictor for cardiovascular events in ESRD

In myocardial infarction, the platelet glycoprotein (GP) IIb of the IIb/IIIa complex is activated, and accelerates platelet aggregation and thrombosis by acting as a receptor for fibrinogen; conversely, inhibition of the GP IIb/IIIa complex has been shown to ameliorate the outcome of cardiac ischemia (4, 8). Our results now indicate that ESRD patients with an increased ITGA2B expression after a two-day dialysis-free interval have an increased risk to develop a cardiovascular event over an observation period of two years. The surface of resting platelets typically contains 50.000 to 80.000 GP IIb/IIIa complexes (43). The 3.24-fold increase in mRNA content suggests that GP IIb/IIIa complexes accumulate on platelets of hemodialysis patients with high cardiovascular risk. Accelerated GP IIb/IIIa-mediated cross-linking of platelets during ischemia may facilitate rapid thrombus formation, and perhaps contributes to the high frequency of sudden cardiac death and unfavorable outcome after myocardial infarction in ESRD patients.

Platelet activation and the formation of platelet-leukocyte aggregates during hemodialysis have been utilized to determine the biocompatibility of a renal replacement modality for several years (32). However, more recently, the presence of platelet-monocyte aggregates has been linked to an increased cardiovascular risk in patients with ESRD irrespective of the dialysis modality, but not to other cell aggregates or certain gene expression products (5). In addition to the increased expression of ITGA2B, two secreted CXC chemokines, pro-platelet basic protein (PPBP, CXCL7) and Platelet Factor 4 (PF4, CXCL4) were up-regulated in patients with high cardiovascular risk. While PBP appears to promote inflammation (14), PF4 plays a role in homeostasis and thrombosis (40). PF4 is an abundant platelet CXC chemokine, released from activated platelets as a 7.8 kDa protein. PF4 forms a complex with heparin that triggers the formation of PF4 antibodies and heparin-induced thrombocytopenia. The present study could further identify GP6, GP9 and PDGFC expression to be linked to an increased cardiovascular risk.

### References

1. Adiguzel, C, Bansal, V, Litinas, E, Cunanan, J, Iqbal, O, Nelson, K, Kannan, M, Hoppensteadt, D & Fareed, J: Increased prevalence of antiheparin platelet factor 4 antibodies in patients may be due to contaminated heparin. Clin Appl Thromb Hemost, 15: 145-51, 2009.
2. Anavekar, NS, McMurray, JJ, Velazquez, EJ, Solomon, SD, Kober, L, Rouleau, JL, White, HD, Nordlander, R, Maggioni, A, Dickstein, K, Zelenkofske, S, Leimberger, JD, Califf, RM & Pfeffer, MA: Relation between renal dysfunction and cardiovascular outcomes after myocardial infarction. N Engl J Med, 351: 1285-95, 2004.
3. Arimura, T, Nakamura, T, Hiroi, S, Satoh, M, Takahashi, M, Ohbuchi, N, Ueda, K, Nouchi, T, Yamaguchi, N, Akai, J, Matsumori, A, Sasayama, S & Kimura, A: Characterization of the human nebulette gene: a polymorphism in an actin-binding motif is associated with nonfamilial idiopathic dilated cardiomyopathy. Hum Genet, 107: 440-51, 2000.
4. Arora, RR & Rai, F: Antiplatelet intervention in acute coronary syndrome. Am J Ther, 16:e29-40, 2009.
5. Ashman, N, Macey, MG, Fan, SL, Azam, U & Yaqoob, MM: Increased platelet-monocyte aggregates and cardiovascular disease in end-stage renal failure patients. Nephrol Dial Transplant, 18: 2088-96, 2003.
6. Beer, PA, Ortmann, CA, Campbell, PJ & Green, AR: Independently acquired biallelic JAK2 mutations are present in a minority of patients with essential thrombocythemia. Blood, 116: 1013-4, 2010.
7. Benjamini, Y & Hochberg, Y: Controlling the false discovery rate: a pratical and powerful approach to multiple testing. J R Stat Soc, 57: 289-295, 1995.
8. Bhatt, DL & Topol, EJ: Current role of platelet glycoprotein IIb/IIIa inhibitors in acute coronary syndromes. JAMA, 284: 1549-58, 2000.
9. Binder, H, Allignol, A, Schumacher, M & Beyersmann, J: Boosting for high-dimensional time-to-event data with competing risks. Bioinformatics, 25: 890-6, 2009.
10. Binder, H & Schumacher, M: Adapting prediction error estimates for biased complexity selection in high-dimensional bootstrap samples. Stat Appl Genet Mol Biol, 7: Article12, 2008.
11. Binder, H & Schumacher, M: Allowing for mandatory covariates in boosting estimation of sparse high-dimensional survival models. BMC Bioinformatics, 9: 14, 2008.
12. Bishop-Bailey, D: PPAR receptor activation: Experimental studies on cardiac structure and function. Curr Opin Investig Drugs, 11: 283-8, 2010.
13. Boldrick, JC, Alizadeh, AA, Diehn, M, Dudoit, S, Liu, CL, Belcher, CE, Botstein, D, Staudt, LM, Brown, PO & Relman, DA: Stereotyped and specific gene expression programs in human innate immune responses to bacteria. Proc Natl Acad Sci U S A, 99:972-7, 2002.
14. Brandt, E, Ludwig, A, Petersen, F & Flad, HD: Platelet-derived CXC chemokines: old players in new games. Immunol Rev, 177: 204-16, 2000.
15. Brezniceanu, ML, Lau, CJ, Godin, N, Chenier, I, Duclos, A, Ethier, J, Filep, JG, Ingelfinger, JR, Zhang, SL & Chan, JS: Reactive oxygen species promote caspase-12 expression and tubular apoptosis in diabetic nephropathy. J Am Soc Nephrol, 21: 943-54, 2010.
16. Carrier, M, Rodger, MA, Fergusson, D, Doucette, S, Kovacs, MJ, Moore, J, Kelton, JG & Knoll, GA: Increased mortality in hemodialysis patients having specific antibodies to the platelet factor 4-heparin complex. Kidney Int, 73: 213-9, 2008.
17. Chan, KE, Lazarus, JM, Thadhani, R & Hakim, RM: Anticoagulant and antiplatelet usage associates with mortality among hemodialysis patients. J Am Soc Nephrol, 20: 872-81, 2009.
18. Chan, KE, Lazarus, JM, Thadhani, R & Hakim, RM: Warfarin use associates with increased risk for stroke in hemodialysis patients with atrial fibrillation. J Am Soc Nephrol, 20: 2223-33, 2009.
19. Chen, YJ, Kodell, R, Sistare, F, Thompson, KL, Morris, S & Chen, JJ: Normalization methods for analysis of microarray gene-expression data. J Biopharm Stat, 13: 57-74, 2003.
20. Chim, CS, Sim, JP, Chan, CC, Kho, BC, Chan, JC, Wong, LG, Law, M, Liang, R & Kwong, YL: Impact of JAK2V617F mutation on thrombosis and myeloid transformation in essential thrombocythemia: a multivariate analysis by Cox regression in 141 patients. Hematology, 15: 187-92, 2010.
21. Coller, BS & Shattil, SJ: The GPIIb/IIIa (integrin alphallbbeta3) odyssey: a technologydriven saga of a receptor with twists, turns, and even a bend. Blood, 112: 3011-25, 2008.
22. Collins, AJ, Foley, RN, Gilbertson, DT & Chen, SC: The state of chronic kidney disease, ESRD, and morbidity and mortality in the first year of dialysis. Clin J Am Soc Nephrol, 4 Suppl 1: S5-11, 2009.
23. de Bie, MK, van Dam, B, Gaasbeek, A, van Buren, M, van Erven, L, Bax, JJ, Schalij, MJ, Rabelink, TJ & Jukema, JW: The current status of interventions aiming at reducing sudden cardiac death in dialysis patients. Eur Heart J, 30: 1559-64, 2009.
24. Dennis, G, Jr., Sherman, BT, Hosack, DA, Yang, J, Gao, W, Lane, HC & Lempicki, RA: DAVID: Database for Annotation, Visualization, and Integrated Discovery. Genome Biol, 4: P3, 2003.
25. Efron, B & Tibshirani, R: On testing the significance of sets of genes. Ann Appl Stat, 1:107-129, 2007.
26. Eisen, MB & Brown, PO: DNA arrays for analysis of gene expression. Methods Enzymol, 303: 179-205, 1999.
27. Fellstrom, BC, Jardine, AG, Schmieder, RE, Holdaas, H, Bannister, K, Beutler, J, Chae, DW, Chevaile, A, Cobbe, SM, Gronhagen-Riska, C, De Lima, JJ, Lins, R, Mayer, G, McMahon, AW, Parving, HH, Remuzzi, G, Samuelsson, O, Sonkodi, S, Sci, D, Suleymanlar, G, Tsakiris, D, Tesar, V, Todorov, V, Wiecek, A, Wuthrich, RP, Gottlow, M, Johnsson, E & Zannad, F: Rosuvastatin and cardiovascular events in patients undergoing hemodialysis. N Engl J Med, 360: 1395-407, 2009.
28. Finck, BN, Han, X, Courtois, M, Aimond, F, Nerbonne, JM, Kovacs, A, Gross, RW & Kelly, DP: A critical role for PPARalpha-mediated lipotoxicity in the pathogenesis of diabetic cardiomyopathy: modulation by dietary fat content. Proc Natl Acad Sci U S A, 100: 1226-31, 2003.
29. Finck, BN, Lehman, JJ, Leone, TC, Welch, MJ, Bennett, MJ, Kovacs, A, Han, X, Gross, RW, Kozak, R, Lopaschuk, GD & Kelly, DP: The cardiac phenotype induced by PPARalpha overexpression mimics that caused by diabetes mellitus. J Clin Invest, 109:121-30, 2002.
30. Fine, J & gray, R: A proportional hazards model for the subdistribution of a competing risk. Am Stat Assoc, 94: 496-509, 1999.
31. Foley, RN, Parfrey, PS & Sarnak, MJ: Epidemiology of cardiovascular disease in chronic renal disease. J Am Soc Nephrol, 9: S 16-23, 1998.
32. Gawaz, MP, Mujais, SK, Schmidt, B, Blumenstein, M & Gurland, HJ: Platelet-leukocyte aggregates during hemodialysis: effect of membrane type. Artif Organs, 23: 29-36, 1999.
33. Gerds, TA & Schumacher, M: Consistent estimation of the expected Brier score in general survival models with right-censored event times. Biom J, 48: 1029-40, 2006.
34. Goodkin, DA, Bragg-Gresham, JL, Koenig, KG, Wolfe, RA, Akiba, T, Andreucci, VE, Saito, A, Rayner, HC, Kurokawa, K, Port, FK, Held, PJ & Young, EW: Association of comorbid conditions and mortality in hemodialysis patients in Europe, Japan, and the United States: the Dialysis Outcomes and Practice Patterns Study (DOPPS). J Am Soc Nephrol, 14: 3270-7, 2003.
35. Herzog, CA, Ma, JZ & Collins, AJ: Poor long-term survival after acute myocardial infarction among patients on long-term dialysis. N Engl J Med, 339: 799-805, 1998.
36. Higuchi, T, Orita, T, Nakanishi, S, Katsuya, K, Watanabe, H, Yamasaki, Y, Waga, I, Nanayama, T, Yamamoto, Y, Munger, W, Sun, HW, Falk, RJ, Jennette, JC, Alcorta, DA, Li, H, Yamamoto, T, Saito, Y & Nakamura, M: Molecular cloning, genomic structure, and expression analysis of MUC20, a novel mucin protein, up-regulated in injured kidney. J Biol Chem, 279: 1968-79, 2004.
37. Himmelfarb, J: The HEMO study - where do we go from here? Curr Opin Nephrol Hypertens, 12: 587-91, 2003.
38. Kanehisa, M & Goto, S: KEGG: kyoto encyclopedia of genes and genomes. Nucleic Acids Res, 28: 27-30, 2000.
39. Kerendi, F, Thourani, VH, Puskas, JD, Kilgo, PD, Osgood, M, Guyton, RA & Lattouf, OM: Impact of heparin-induced thrombocytopenia on postoperative outcomes after cardiac surgery. Ann Thorac Surg, 84: 1548-53; discussion 1554-5, 2007.
40. Kowalska, MA, Rauova, L & Poncz, M: Role of the platelet chemokine platelet factor 4 (PF4) in hemostasis and thrombosis. Thromb Res, 125: 292-6, 2010.
41. Krane, V, Berger, M, Lilienthal, J, Winkler, K, Schambeck, C & Wanner, C: Antibodies to platelet factor 4-heparin complex and outcome in hemodialysis patients with diabetes. Clin J Am Soc Nephrol, 5: 874-81, 2010.
42. Kriegova, E, Arakelyan, A, Fillerova, R, Zatloukal, J, Mrazek, F, Navratilova, Z, Kolek, V, du Bois, RM & Petrek, M: PSMB2 and RPL32 are suitable denominators to normalize gene expression profiles in bronchoalveolar cells. BMC Mol Biol, 9: 69, 2008.
43. Lefkovits, J, Plow, EF & Topol, EJ: Platelet glycoprotein IIb/IIIa receptors in cardiovascular medicine. N Engl J Med, 332: 1553-9, 1995.
44. Li, G, Zhang, H, Lv, J, Hou, P & Wang, H: Tandem repeats polymorphism of MUC20 is an independent factor for the progression of immunoglobulin A nephropathy. Am J Nephrol, 26: 43-9, 2006.
45. London, GM: Cardiovascular disease in chronic renal failure: pathophysiologic aspects. Semin Dial, 16: 85-94, 2003.
46. Maree, A & Fitzgerald, DJ: Glycoprotein IIb/IIIa antagonists in acute coronary syndromes: where are we now? Semin Vasc Med, 3: 385-90, 2003.
47. McCarthy, N, McCarron, SL & Langabeer, SE: Prevalence of the JAK2 V617F and MPL mutations in stroke, abdominal and peripheral venous thrombosis. Acta Haematol, 124:160-1, 2010.
48. Panagia, M, Gibbons, GF, Radda, GK & Clarke, K: PPAR-alpha activation required for decreased glucose uptake and increased susceptibility to injury during ischemia. Am J Physiol Heart Circ Physiol, 288: H2677-83, 2005.
49. Pena de la Vega, L, Miller, RS, Benda, MM, Grill, DE, Johnson, MG, McCarthy, JT & McBane, RD, 2nd: Association of heparin-dependent antibodies and adverse outcomes in hemodialysis patients: a population-based study. Mayo Clin Proc, 80: 995-1000, 2005.
50. Pepe, MS & Mori, M: Kaplan-Meier, marginal or conditional probability curves in summarizing competing risks failure time data? Stat Med, 12: 737-51, 1993.
51. Purevjav, E, Varela, J, Morgado, M, Kearney, DL, Li, H, Taylor, MD, Arimura, T, Moncman, CL, McKenna, W, Murphy, RT, Labeit, S, Vatta, M, Bowles, NE, Kimura, A, Boriek, AM & Towbin, JA: Nebulette mutations are associated with dilated cardiomyopathy and endocardial fibroelastosis. J Am Coll Cardiol, 56: 1493-502, 2010.
52. Sambandam, N, Morabito, D, Wagg, C, Finck, BN, Kelly, DP & Lopaschuk, GD: Chronic activation of PPARalpha is detrimental to cardiac recovery after ischemia. Am J Physiol Heart Circ Physiol, 290: H87-95, 2006.
53. Sarnak, MJ, Levey, AS, Schoolwerth, AC, Coresh, J, Culleton, B, Hamm, LL, McCullough, PA, Kasiske, BL, Kelepouris, E, Klag, MJ, Parfrey, P, Pfeffer, M, Raij, L, Spinosa, DJ & Wilson, PW: Kidney disease as a risk factor for development of cardiovascular disease: a statement from the American Heart Association Councils on Kidney in Cardiovascular Disease, High Blood Pressure Research, Clinical Cardiology, and Epidemiology and Prevention. Hypertension, 42: 1050-65, 2003.
54. Sazawal, S, Bajaj, J, Chikkara, S, Jain, S, Bhargava, R, Mahapatra, M & Saxena, R: Prevalence of JAK2 V617F mutation in Indian patients with chronic myeloproliferative disorders. Indian J Med Res, 132: 423-7, 2010.
55. Villanueva, MP, Aiyer, AR, Muller, S, Pletcher, MT, Liu, X, Emanuel, B, Srivastava, D & Reeves, RH: Genetic and comparative mapping of genes dysregulated in mouse hearts lacking the Hand2 transcription factor gene. Genomics, 80: 593-600, 2002.
56. Wanner, C, Krane, V, Marz, W, Olschewski, M, Mann, JF, Ruf, G & Ritz, E: Atorvastatin in patients with type 2 diabetes mellitus undergoing hemodialysis. N Engl J Med, 353:238-48, 2005.
57. Weinhandl, ED, Foley, RN, Gilbertson, DT, Arneson, TJ, Snyder, JJ & Collins, AJ: Propensity-matched mortality comparison of incident hemodialysis and peritoneal dialysis patients. J Am Soc Nephrol, 21: 499-506, 2010.
58. Weston, H, Cowell, V, Grimmett, K, Saal, R, Jones, M, Mills, T, Gill, D, Marlton, P, Bird, R & Mollee, P: Prognostic Utility Of Spontaneous Erythroid Colony Formation And JAK2 Mutational Analysis For Thrombotic Events In Essential Thrombocythaemia. Intern Med J, 2010.
59. Yang, YH, Dudoit, S, Luu, P, Lin, DM, Peng, V, Ngai, J & Speed, TP: Normalization for cDNA microarray data: a robust composite method addressing single and multiple slide systematic variation. Nucleic Acids Res, 30: e15, 2002.

## Claims

1. A method for predicting the risk of a cardiovascular event in a subject suffering from end-stage renal disease, comprising determining in a sample from said subject the expression level of at least one gene selected from the group consisting of PPBP, GP6, PF4, GP9, PDGFC and ITGA2B.

2. A method for predicting the mortality risk after a cardiovascular event in a subject suffering from end-stage renal disease, comprising determining in a sample from said subject the expression level of at least one gene selected from the group consisting of PPBP, GP6, PF4, GP9, PDGFC and ITGA2B.

3. A method for monitoring the impact of a treatment administered to a subject suffering from end-stage renal disease on the risk of a cardiovascular event, comprising determining in a sample from said subject the expression level of at least one gene selected from the group consisting of PPBP, GP6, PF4, GP9, PDGFC and ITGA2B.

4. A method for predicting a cardiovascular disease or for monitoring the progression of a cardiovascular disease in a subject suffering from end-stage renal disease, comprising determining in a sample from said subject the expression level of at least one gene selected from the group consisting of PPBP, GP6, PF4, GP9, PDGFC and ITGA2B.

5. The method according to any one of claims 1 to 4, wherein the subject has not previously shown symptoms of cardiovascular disease and/or has not previously been diagnosed with a cardiovascular disease.

6. A method for predicting the risk of a cardiovascular event in a subject who has not previously shown symptoms of cardiovascular disease and/or has not previously been diagnosed with a cardiovascular disease, comprising determining in a sample from said subject the expression level of at least one gene selected from the group consisting of PPBP, GP6, PF4, GP9, PDGFC and ITGA2B.

7. The method of any one of claims 1 to 6, wherein the subject undergoes hemodialysis.

8. The method of any one of claims 1-7, wherein the step of determining the expression level of said at least one gene comprises determining the amount of at least one mRNA in said sample, wherein the cDNA sequence of said at least one mRNA comprises a nucleotide sequence selected from the group consisting of SEQ ID NO:1, SEQ ID NO:2, SEQ ID NO:3, SEQ ID NO:4, SEQ ID NO:5, SEQ ID NO:6, SEQ ID NO:7, and SEQ ID NO:8.

9. The method of any one of claims 1-7, wherein the step of determining the expression of said at least one gene comprises determining the amount of at least one polypeptide in said sample, wherein said at least one polypeptide comprises an amino acid sequence selected from the group consisting of SEQ ID NO:9, SEQ ID NO:10, SEQ ID NO:11 SEQ ID NO:12, SEQ ID NO:13, SEQ ID NO:14, SEQ ID NO:15, SEQ ID NO:16, SEQ ID NO:17, SEQ ID NO:18, and SEQ ID NO:19.

10. A method according to any one of claims 1 to 9, wherein the expression levels of at least two, preferably at least three different genes selected from the group consisting of PPBP, GP6, PF4, GP9, PDGFC and ITGA2B are determined.

11. The method according to any one of claims 1 to 10, wherein the sample is a blood sample.

12. The method of any one of claims 1 to 11, wherein the expression level of the PPBP gene is determined.

13. The method of any one of claims 1 to 12, wherein the expression level of the ITGA2B gene is determined.

14. The method of any one of claims 1 to 13, wherein the expression level of the PF4 gene is determined.

15. The use of at least one gene selected from the group consisting of PPBP, GP6, PF4, GP9, PDGFC and ITGA2B, or of at least one expression product thereof as a biomarker for predicting the risk of a cardiovascular event or the mortality risk after a cardiovascular event in a subject suffering from end-stage renal disease.
